# EUROPEAN PATENT APPLICATION

(11) **EP 4 834 331 A1**
(43) Date of publication of application: **04.11.2026**
(21) Application number: 24911502.3
(22) Date of filing: 27.12.2024
(51) Int. Cl.: A61K 9/20, A61K 9/28, A61K 31/427, A61K 31/506, C07D 417/12, A61P 11/00, A61P 11/06, A61P 11/14, A61P 13/00, A61P 13/02, A61P 13/08, A61P 13/10, A61P 15/00, A61P 25/00, A61P 25/04, A61P 25/02, A61P 25/06, A61P 29/00

(54) **SOLID DISPERSION OF P2X3 RECEPTOR ANTAGONIST AND PREPARATION PROCESS THEREOF**

(30) Priority: 29.12.2023 CN 202311872796; 23.12.2024 CN 202411907674
(71) Applicant: Hubei Bio-Pharmaceutical Industrial Technological Institute Inc., Wuhan, Hubei 430075 (CN)
(72) Inventor: SONG, Haoyuan, Wuhan, Hubei 430075 (CN); YANG, Yiping, Wuhan, Hubei 430075 (CN); CAO, Xueteng, Wuhan, Hubei 430075 (CN); HUANG, Xiang, Wuhan, Hubei 430075 (CN); XU, Zebin, Wuhan, Hubei 430075 (CN); HU, Hui, Wuhan, Hubei 430075 (CN); JIN, Ying, Wuhan, Hubei 430075 (CN); ZHANG, Bo, Wuhan, Hubei 430075 (CN)
(74) Representative: Gille Hrabal Partnerschaftsgesellschaft mbB Patentanwälte
(86) International application number: PCT/CN2024/143259
(87) International publication number: WO 2025/140586

(57) **Abstract**

Provided is a solid dispersion containing a P2X3 receptor antagonist compound represented by formula I, which can increase the solubility of the compound shown in formula I, and improve the dissolution rate and bioavailability of the drug, ensuring the therapeutic effect of the drug. Meanwhile, the solid dispersion has good stability. During the storage process, the solid dispersion does not experience crystal form transformation and does not exhibit a significant increase in related substances, which improves the safety of the clinical use of the drug.

## Description

The present application claims priority to the following prior applications:
the prior application with the patent application No. 202311872796.5 and entitled " A solid dispersion of P2X3 receptor antagonist and preparation process thereof" filed with China National Intellectual Property Administration on December 29, 2023; and
the prior application with the patent application No. 202411907674.X, and entitled " A solid dispersion of P2X3 receptor antagonist and preparation process thereof" filed with China National Intellectual Property Administration on December 23, 2024, which are incorporated herein by reference in their entireties.

### Technical Field

The present disclosure belongs to the field of pharmaceutical preparations, and in particular, relates to a solid dispersion of P2X3 receptor antagonist and preparation process thereof.

### Background

P2X3 is a subunit of ATP-gated ion channels, and belongs to a member of the purinergic P2X family, which is both expressed in the airway and central nerve endings. A number of clinical studies have confirmed that P2X3 receptor antagonist can significantly reduce cough frequency, cough urge and cough severity in patients with refractory chronic cough. The most common adverse reaction is hypogeusia. The incidence of taste disorder can be reduced by lowering the drug dose and selecting P2X3 receptor antagonist with higher selectivity. At present, a number of clinical trials on P2X3 are underway. P2X3 receptor antagonist is expected to become the most promising therapeutic drugs for refractory chronic cough in the future.

Gefapixant, the first selective P2X3 receptor antagonist for the same target, was approved for marketing in Japan in January 2022, and with a New Drug Application (NDA) planned to be submitted to the FDA and EMA in 2023. Its clinical adverse reactions include taste loss or hypogeusia, xerostomia, nausea and the like. Taste disorder may be associated with the effect of Gefapixant on P2X2/3 receptors. Thus, it is necessary to develop highly selective P2X3 receptor antagonist to reduce the adverse reactions of taste loss or taste disorder. In addition, a number of selective P2X3 receptor antagonist are under clinical development: Camlipixant (Phase III) by Bellus, Eliapixant (Phase IIb) by Bayer, Sivopixant by Shionogi (Phase III planned), and four candidates in China at Phase I-II stage, all with favorable target safety profile. Compared with the marketed Gefapixant, the compound represented by Formula I, a specific P2X3 receptor antagonist involved in the present disclosure, can significantly reduce the occurrence of taste loss and is intended for the treatment of refractory chronic cough.

However, during the research process, the inventors of the present disclosure found that, the compound represented by Formula I has poor solubility: its saturated solubility is less than 0.02 mg/mL in media with a pH value ranging from 1.0 to 6.8, and its dissolution rate is less than 10% in media of pH 1.2, pH 4.5, pH 6.8 and pure water. In addition, the bulk drug has poor thermokinetic stability, being prone to crystallization during storage and thus exhibiting poor stability overall. Therefore, there is a need to provide a novel preparation to address the problems of poor solubility and poor stability of the compound represented by Formula I.

### Summary of the Invention

To ameliorate the aforesaid technical problems, the present disclosure provides a solid dispersion of P2X3 receptor antagonist (the compound represented by Formula I). The solid dispersion can increase the solubility of the compound represented by Formula I, and significantly improve the dissolution rate and bioavailability of the drug. Meanwhile, the solid dispersion has good stability. During the storage process, the solid dispersion does not experience crystal form transformation.

The present disclosure provides a solid dispersion (SD) of a P2X3 receptor antagonist. The solid dispersion comprising: a P2X3 receptor antagonist and a pharmaceutically acceptable carrier;
wherein, the P2X3 receptor antagonist is selected from a compound represented by Formula I, or a tautomer, a stereoisomer, a hydrate, a solvate, a pharmaceutically acceptable salt, or a prodrug thereof: 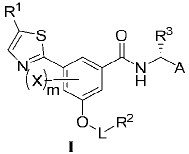
wherein:
   R¹ is independently selected from halogen, C₃-C₆ cycloalkyl, unsubstituted C₁-C₄ alkyl, or C₁-C₄ alkyl substituted with 1 to 5 identical or different halogen atoms;
   X is halogen;
   m is an integer selected from 1, 2, or 3;
   L is -(CH₂)ₙ-, where n is an integer selected from 0, 1, or 2;
   R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
      the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
      R³ is independently selected from hydrogen, unsubstituted C₁-C₄ alkyl, or C₁-C₄ alkyl substituted with 1 to 5 identical or different halogen atoms;
      R⁴ is independently selected from hydrogen or C₁-C₄ alkyl;
      R^{b} and R^{c} are independently selected from hydrogen or C₁-C₄ alkyl; and
      A is independently unsubstituted or R^{e}-substituted 5-10-membered heteroaryl, wherein the R^{e}-substituted 5-10-membered heteroaryl has one or more R^{e} substituents, each R^{e} substituent being independently selected from halogen, unsubstituted C₁-C₃ alkyl, C₁-C₃ alkyl substituted with 1 to 5 identical or different halogen atoms, unsubstituted -O-(C₁-C₃ alkyl), or -O-(C₁-C₃ alkyl) substituted with 1 to 5 identical or different halogen atoms; when the one or more R^{e} substituents include a plurality of R^{e} substituents, the plurality of R^{e} substituents is identical or different.

In an embodiment of the present disclosure, the solid dispersion is an amorphous solid dispersion.

In an embodiment of the present disclosure, the P2X3 receptor antagonist is present in an amorphous form.

In an embodiment of the present disclosure, the pharmaceutically acceptable carrier is a pharmaceutically acceptable polymeric carrier.

In an embodiment of the present disclosure, the pharmaceutically acceptable carrier is selected from any one or more of the following:
1) a cellulose derivative carrier, for example, a hypromellose E5 (HPMC-E5), a hypromellose acetate succinate LF (HPMC-AS-LF), a hydroxypropyl cellulose (HPC), a hypromellose phthalate (HPMCP), and the like;
2) a polyacrylic resin carrier, for example, a Eudragit L100-55, a Eudragit S100, a Eudragit L100;
3) a vinyl polymer carrier, for example, a polyvinylcaprolactam-polyvinyl acetate-polyethylene glycol graft copolymer (Soluplus), a copovidone VA64, a copovidone S630, and the like.

In an embodiment of the present disclosure, the pharmaceutically acceptable carrier is selected from any one or more of the following: hypromellose (HPMC), copovidone (PVP), hypromellose acetate succinate (HPMC-AS), polymethacrylate (Eudragit), and polyvinylcaprolactam-polyvinyl acetate-polyethylene glycol graft copolymer (Soluplus). Preferably, it is selected from any one or more of the following: hypromellose (HPMC), hypromellose acetate succinate (HPMC-AS), and polymethacrylate (Eudragit).

In an embodiment of the present disclosure, the pharmaceutically acceptable carrier is selected from any one or more of the following: hypromellose E5 (HPMC-E5), copovidone VA64 (PVP VA64), hypromellose acetate succinate LF (HPMC-AS-LF), Eudragit L100-55, and polyvinylcaprolactam-polyvinyl acetate-polyethylene glycol graft copolymer (Soluplus). Preferably, it is selected from any one or more of the following: hypromellose E5 (HPMC-E5), hypromellose acetate succinate LF (HPMC-AS-LF), and Eudragit L100-55.

In an embodiment of the present disclosure, the mass ratio of the P2X3 receptor antagonist to the pharmaceutically acceptable carrier is 1:(0.1-10), preferably 1:(0.5-5), for example, 1:0.5, 1:1, 1:2, 1:3, 1:4 or 1:5.

In an embodiment of the present disclosure, the solid dispersion comprises the following components:
the P2X3 receptor antagonist: 10wt%-60wt%
the pharmaceutically acceptable carrier: 40wt%-90wt%.

In an embodiment of the present disclosure, the solid dispersion comprises the following components:
the P2X3 receptor antagonist: 20wt%-55wt%
the pharmaceutically acceptable carrier: 45wt%-80wt%.

In an embodiment of the present disclosure, the solid dispersion comprises the following components:
the P2X3 receptor antagonist: 25wt%-50wt% (e.g., 25wt%, 33wt%, 33.3wt%, 50wt%)
the pharmaceutically acceptable carrier: 50wt%-75wt% (e.g., 50wt%, 67wt%, 66.7wt%, 75wt%).

In an embodiment of the present disclosure, the solid dispersion comprises the following components: the P2X3 receptor antagonist and the pharmaceutically acceptable carrier, wherein the content of the pharmaceutically acceptable carrier is not less than 66.7wt%.

In an embodiment of the present disclosure, the solid dispersion comprises the following components: the P2X3 receptor antagonist and the pharmaceutically acceptable carrier, wherein the content of the pharmaceutically acceptable carrier is not less than 75wt%.

In an embodiment of the present disclosure, the solid dispersion comprises the following components:
the P2X3 receptor antagonist: 25 wt%, 33.3 wt%, 50 wt%
the pharmaceutically acceptable carrier: 50 wt%, 66.7 wt%, 75 wt%.

The pharmaceutically acceptable carrier is selected from hypromellose E5 (HPMC-E5), hypromellose acetate succinate LF (HPMC-AS-LF), and Eudragit L100-55.

In an embodiment of the present disclosure, the solid dispersion comprises the following components:
the P2X3 receptor antagonist: 25 wt%
the pharmaceutically acceptable carrier: 75 wt%.

The pharmaceutically acceptable carrier is selected from hypromellose E5 (HPMC-E5), hypromellose acetate succinate LF (HPMC-AS-LF), and Eudragit L100-55.

In an embodiment of the present disclosure, the solid dispersion comprises the following components:
the P2X3 receptor antagonist: 33.3 wt%
the pharmaceutically acceptable carrier: 66.7 wt%.

The pharmaceutically acceptable carrier is selected from hypromellose E5 (HPMC-E5), hypromellose acetate succinate LF (HPMC-AS-LF), and Eudragit L100-55.

In an embodiment of the present disclosure, the solid dispersion comprises the following components: the P2X3 receptor antagonist and hypromellose acetate succinate LF, wherein the content of hypromellose acetate succinate LF is not less than 75%.

In an embodiment of the present disclosure, the solid dispersion comprises the following components:
the P2X3 receptor antagonist: 25 wt%
the hypromellose acetate succinate LF: 75 wt%.

In an embodiment of the present disclosure, the solid dispersion comprises the following components:
the P2X3 receptor antagonist and hypromellose acetate succinate LF, wherein the content of hypromellose acetate succinate LF is not less than 66.7 wt%.

In an embodiment of the present disclosure, the solid dispersion comprises the following components:
the P2X3 receptor antagonist: 33.3 wt%
hypromellose acetate succinate LF: 66.7 wt%.

In an embodiment of the present disclosure, the solid dispersion optionally further comprises a solvent.

In an embodiment of the present disclosure, the solvent is a volatile solvent, which volatilizes during the preparation of the solid dispersion.

In an embodiment of the present disclosure, the solvent is selected from any one or more of the following: dichloromethane, ethanol, acetone, and methanol; preferably acetone, or a mixed solvent of dichloromethane and methanol.

In an embodiment of the present disclosure, the solid dispersion optionally further comprises any one or more of the following: a sweetener, a flavoring agent, a colorant, a surfactant, a filler, a lubricant, a disintegrant, a disintegration accelerator, a recrystallization inhibitor, a defoamer, an antioxidant and a pH regulator.

In a preferred embodiment of the present disclosure, the compound represented by Formula I, or the tautomer, the stereoisomer, the hydrate, the solvate, the pharmaceutically acceptable salt, or the prodrug thereof is: 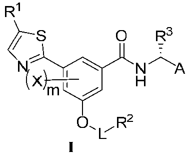 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
X is halogen;
m is an integer selected from 1, 2, or 3;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents each independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
   R³ is independently selected from hydrogen, unsubstituted C₁-C₄ alkyl, or C₁-C₄ alkyl substituted with 1 to 5 identical or different halogen atoms;
   R⁴ is independently selected from hydrogen or C₁-C₄ alkyl;
   R^{b} and R^{c} are independently selected from hydrogen or C₁-C₄ alkyl; and
   A is independently unsubstituted or R^{e}-substituted 5-10-membered heteroaryl, wherein the R^{e}-substituted 5-10-membered heteroaryl has one or more R^{e} substituents, each R^{e} substituent being independently selected from halogen, unsubstituted C₁-C₃ alkyl, C₁-C₃ alkyl substituted with 1 to 5 identical or different halogen atoms, or unsubstituted -O-(C₁-C₃ alkyl), or -O-(C₁-C₃ alkyl) substituted with 1 to 5 identical or different halogen atoms; when the one or more R^{e} substituents include a plurality of R^{e} substituents, the plurality of R^{e} substituents is identical or different.

In a preferred embodiment of the present disclosure, when R¹ is halogen, the halogen is F, Cl, Br, or I, and preferably Cl.

In a preferred embodiment of the present disclosure, when R¹ is the unsubstituted C₁-C₄ alkyl, the C₁-C₄ alkyl is methyl, ethyl, n-propyl, or isopropyl, and preferably methyl or ethyl.

In a preferred embodiment of the present disclosure, when R¹ is the C₁-C₄ alkyl substituted with 1 to 5 identical or different halogen atoms, the C₁-C₄ alkyl is methyl, ethyl, n-propyl, or isopropyl, and preferably methyl or ethyl.

In a preferred embodiment of the present disclosure, when R¹ is the C₁-C₄ alkyl substituted with 1 to 5 identical or different halogen atoms, the halogen atoms are F, Cl, Br, or I, and preferably Cl or F.

In a preferred embodiment of the present disclosure, when X is halogen, the halogen is one of F or Cl.

In a preferred embodiment of the present disclosure, m is an integer selected from 1, 2 or 3, and preferably 1.

In a preferred embodiment of the present disclosure, when L is -(CH₂)ₙ-, n is an integer of 0, 1, or 2, and preferably n is 0 or 1.

In a preferred embodiment of the present disclosure, when R² is the unsubstituted or R^{a}-substituted C₁-C₆ alkyl, the C₁-C₆ alkyl is C₁-C₄ alkyl, and preferably sec-butyl.

In a preferred embodiment of the present disclosure, when R² is the unsubstituted or R^{a}-substituted C₁-C₆ alkyl, the C₁-C₆ alkyl is preferably methyl, ethyl, n-propyl, isopropyl, n-butyl, isobutyl, sec-butyl, or tert-butyl.

In a preferred embodiment of the present disclosure, when R² is the unsubstituted or R^{a}-substituted C₃-C₇ cycloalkyl, the C₃-C₇ alkyl is cyclopropyl, cyclobutyl, cyclopentyl, or cyclohexyl, and preferably cyclopropyl.

In a preferred embodiment of the present disclosure, when R² is the unsubstituted or R^{a}-substituted 4-7-membered heterocycloalkyl, the 4-7-membered heterocycloalkyl is 4-membered, 5-membered, or 6-membered heterocycloalkyl.

In a preferred embodiment of the present disclosure, when R² is the unsubstituted or R^{a}-substituted 4-7-membered heterocycloalkyl, the 4-7-membered heterocycloalkyl has one or more heteroatoms selected from N, S, O, or P, and preferably selected from N or O.

In a preferred embodiment of the present disclosure, when R² is the unsubstituted or R^{a}-substituted 4-7-membered heterocycloalkyl, the 4-7-membered heterocycloalkyl has 1-3 heteroatoms, and preferably 1 or 2 heteroatoms.

In a preferred embodiment of the present disclosure, when R² is the unsubstituted or R^{a}-substituted 4-7-membered heterocycloalkyl, R² has one to three R^{a} substituents, and preferably one R^{a} substituent.

In a preferred embodiment of the present disclosure, R^{a} is hydroxyl.

In a preferred embodiment of the present disclosure, when R^{a} is halogen, the halogen is F, Cl, Br, or I, and preferably F or Cl.

In a preferred embodiment of the present disclosure, when R^{a} is C₁-C₆ alkyl, the C₁-C₆ alkyl is C₁-C₃ alkyl, and preferably methyl.

In a preferred embodiment of the present disclosure, when R^{a} is the deuterated C₁-C₆ alkyl, the C₁-C₆ alkyl is deuterated C₁-C₃ alkyl, and preferably 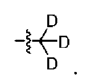 .

In a preferred embodiment of the present disclosure, when R^{a} is -(C₁-C₆ alkylene)-OH, the C₁-C₆ alkylene is C₁-C₄ alkylene, preferably methylene, ethylene, n-propylene, or isopropylene, and more preferably methylene.

In a preferred embodiment of the present disclosure, R³ is hydrogen.

In a preferred embodiment of the present disclosure, when R³ is the unsubstituted C₁-C₄ alkyl, the C₁-C₄ alkyl is methyl, ethyl, n-propyl, or isopropyl, and preferably methyl.

In a preferred embodiment of the present disclosure, when A is the R^{e}-substituted 5-10-membered heteroaryl, the 5-10-membered heteroaryl is 6-membered heteroaryl, and preferably pyrimidinyl or pyridazinyl.

In a preferred embodiment of the present disclosure, when A is the R^{e}-substituted 5-10-membered heteroaryl, A is mono-substituent.

In a preferred embodiment of the present disclosure, when A is the R^{e}-substituted 5-10-membered heteroaryl, the R^{e} substituent is C₁-C₃ alkyl substituted with 1 to 5 identical or difference halogen atoms, and preferably trifluoromethyl.

In a preferred embodiment of the present disclosure, when A is the R^{e}-substituted 5-10-membered heteroaryl, the R^{e} substituent is unsubstituted C₁-C₃ alkyl, and preferably methyl.

In a preferred embodiment of the present disclosure, -L-R² is selected from

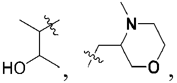

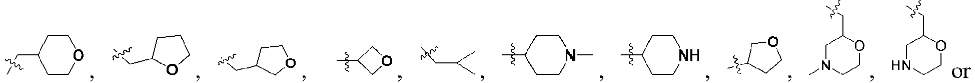

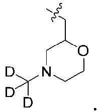

In a preferred embodiment of the present disclosure, -L-R² is

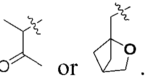

In a preferred embodiment of the present disclosure, -L-R² is selected from

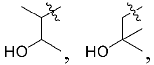

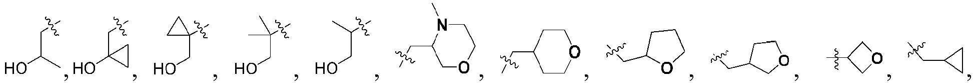

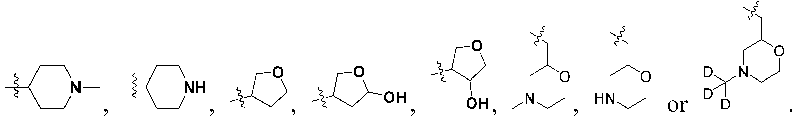

In a preferred embodiment of the present disclosure, R¹ is methyl, ethyl, or Cl.

In a preferred embodiment of the present disclosure, -L-R² is selected from

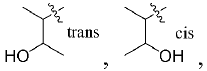

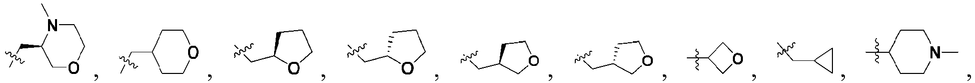

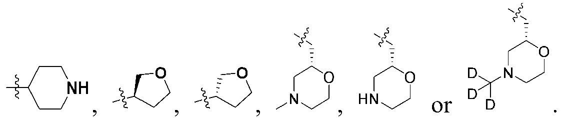

In a preferred embodiment of the present disclosure, -L-R² is selected from

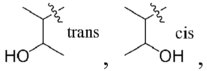

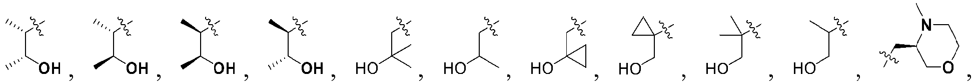

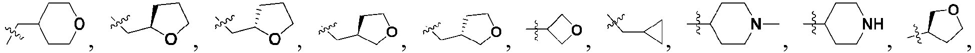

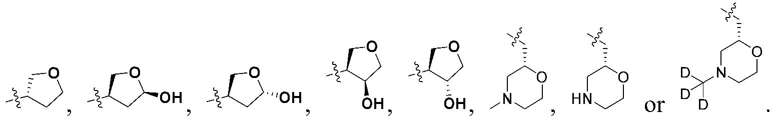

In a preferred embodiment of the present disclosure, R³ is methyl or hydrogen.

In a preferred embodiment of the present disclosure, A is

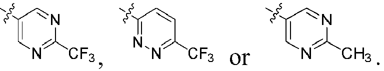

In a preferred embodiment of the present disclosure, the compound represented by Formula I, or the tautomer, the stereoisomer, the hydrate, the solvate, the pharmaceutically acceptable salt, or the prodrug thereof is: 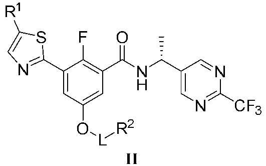 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
   R⁴ is independently selected from hydrogen or C₁-C₄ alkyl;
   R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 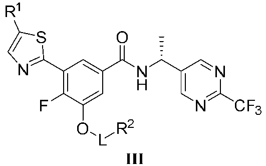 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
   R⁴ is independently selected from hydrogen or C₁-C₄ alkyl;
   R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 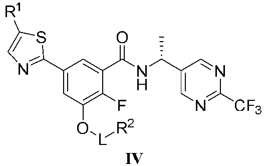 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
   R⁴ is independently selected from hydrogen or C₁-C₄ alkyl;
   R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 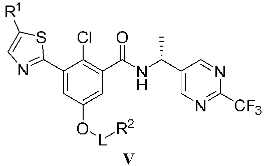 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
   R⁴ is independently selected from hydrogen or C₁-C₄ alkyl;
   R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 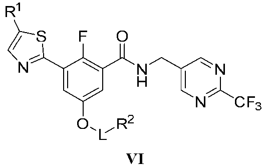 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
   R⁴ is independently selected from hydrogen or C₁-C₄ alkyl;
   R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 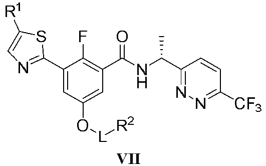 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
   R⁴ is independently selected from hydrogen or C₁-C₄ alkyl;
   R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 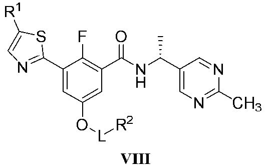 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
   R⁴ is independently selected from hydrogen or C₁-C₄ alkyl;
   R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is selected from:

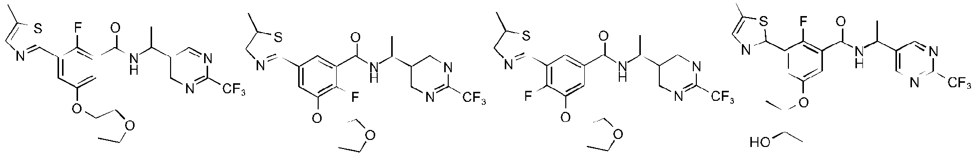

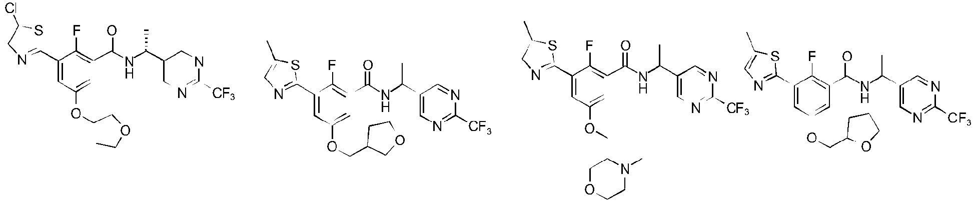

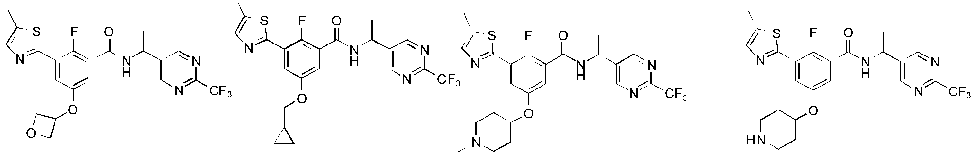

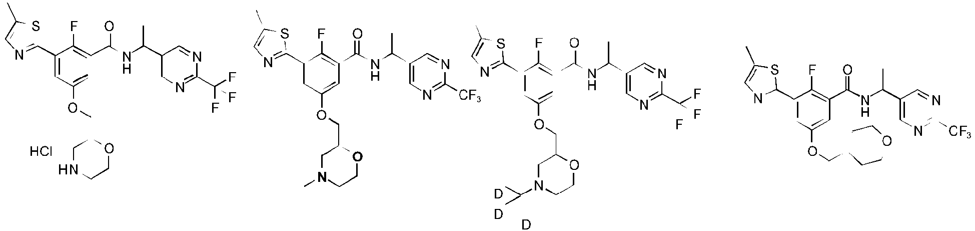

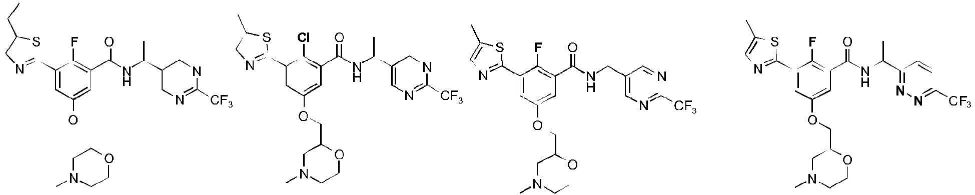

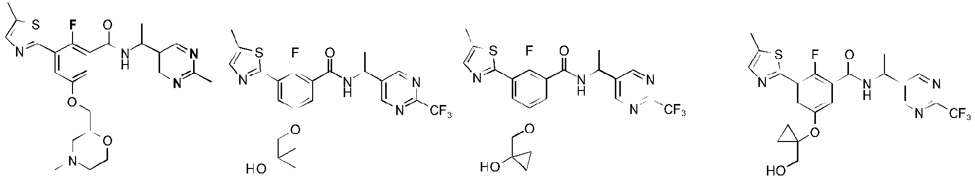

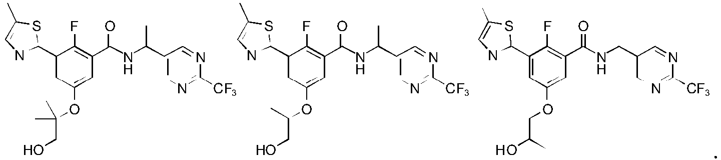

In a preferred embodiment of the present disclosure, the compound represented by Formula I, or the tautomer, the stereoisomer, the hydrate, the solvate, the pharmaceutically acceptable salt, or the prodrug thereof is selected from:

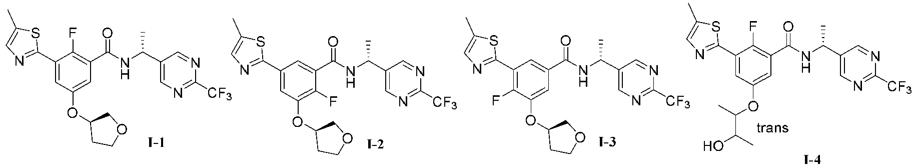

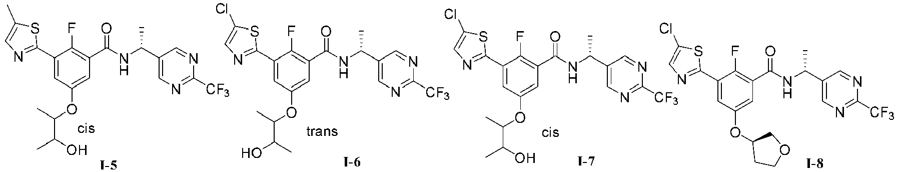

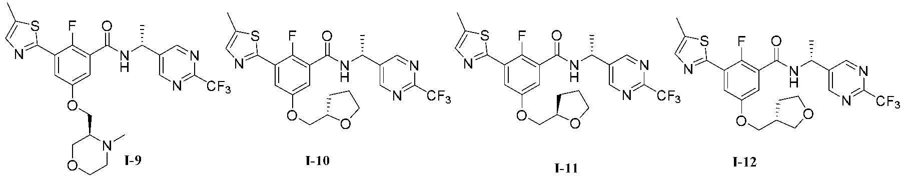

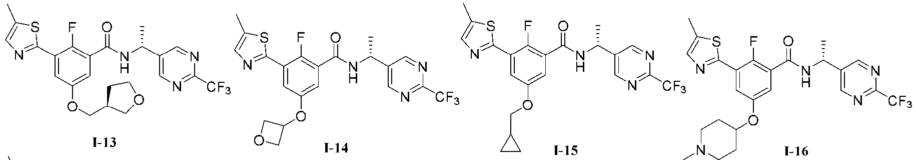

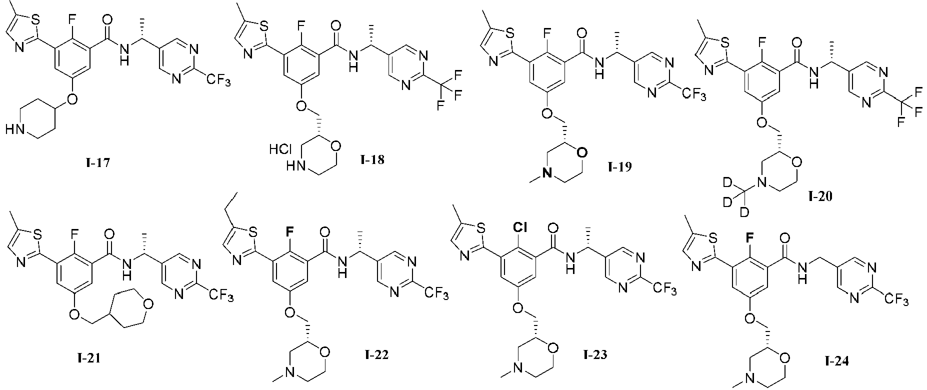

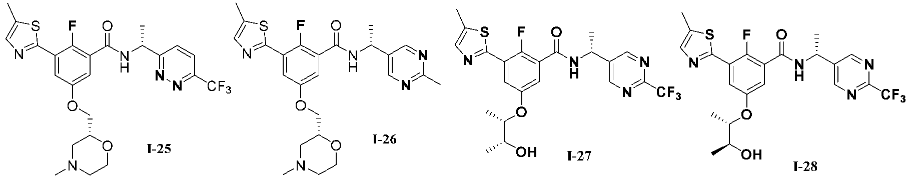

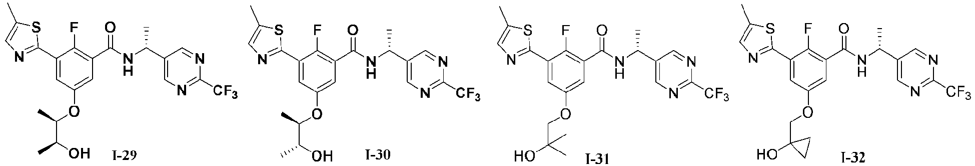

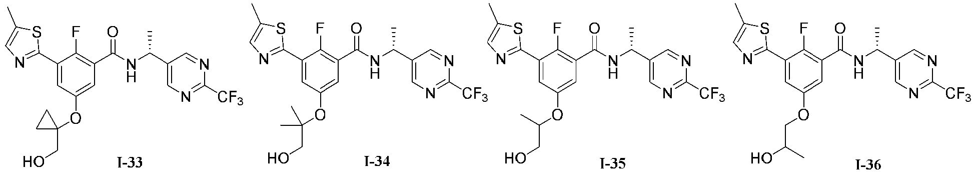

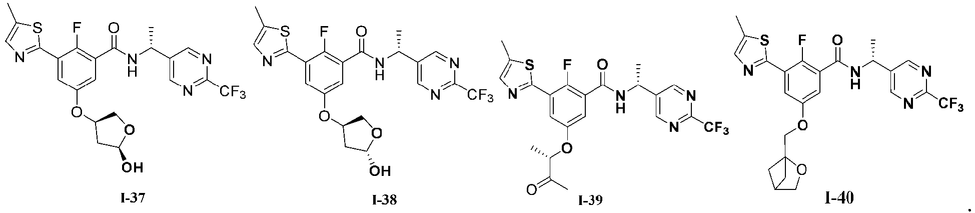

In an embodiment of the present disclosure, the P2X3 receptor antagonist is selected from a compound represented by Formula A, or a tautomer, a stereoisomer, a hydrate, a solvate, a pharmaceutically acceptable salt, or a prodrug thereof:

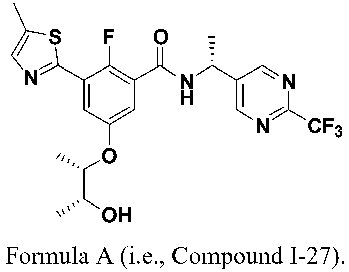

The present disclosure also provides a process for preparing the aforementioned solid dispersion, which comprises the following steps:
(1) adding components to a solvent and stirring to dissolve;
(2) subjecting the dissolved solution to a drying treatment to obtain the solid dispersion.

In an embodiment of the present disclosure, in step (1), the process is specifically: first adding the pharmaceutically acceptable carrier to the solvent and stirring to dissolve; then adding the P2X3 receptor antagonist and continuing to stir to dissolve.

In an embodiment of the present disclosure, in step (2), the drying treatment method is spray drying.

In an embodiment of the present disclosure, in step (2), the process is specifically: performing pressure filtration on the dissolved solution into a transfer tank, then drying the pressure-filtered solution to obtain the solid dispersion;
preferably, performing pressure filtration into the transfer tank through a precision filter with a specific pore size.

In an embodiment of the present disclosure, after the drying treatment in step (2), the dried solid dispersion is optionally further subjected to reduced pressure drying.

In an embodiment of the present disclosure, in step (1), the stirring time is not less than 60min (e.g., not less than 120min); preferably 60-120min or 120-180min.

In an embodiment of the present disclosure, in step (2), the pore size of the precision filter is 1-22µm, preferably 5µm.

In an embodiment of the present disclosure, in step (2), the feeding frequency of the spray drying is 5Hz -50 Hz, preferably 10Hz - 20Hz;
and/or, an atomization pressure is 0.1MPa - 1.0MPa, preferably 0.1MPa - 0.2MPa;
and/or, an inlet air temperature is 60°C-120°C, preferably 85°C-110°C;
and/or, an outlet air temperature is 40°C-70°C, preferably 50°C-55°C.

The present disclosure also provides a pharmaceutical composition, which comprises the aforementioned solid dispersion and a pharmaceutically acceptable carrier, diluent or excipient.

In an embodiment of the present disclosure, the dosage form of the pharmaceutical composition is selected from solid or liquid dosage forms, for example, powders, granules, pills, tablets, sachets, capsules, dragees, chewable tablets, effervescent tablets, dispersible tablets, troches, melts, solutions, suspensions or emulsions, and the like.

According to specific examples of the present disclosure, the pharmaceutical composition is mixed with the pharmaceutically acceptable carrier, diluent or excipient to prepare a pharmaceutical preparation suitable for oral or parenteral administration. The administration methods include, but are not limited to, intradermal, intramuscular, intraperitoneal, intravenous, subcutaneous, intranasal and oral routes.

The present disclosure also provides a pharmaceutical preparation, which comprises the aforementioned solid dispersion and a pharmaceutically acceptable carrier, diluent or excipient.

In an embodiment of the present disclosure, the pharmaceutical preparation is selected from solid or liquid dosage forms, for example, powders, granules, pills, tablets, sachets, capsules, dragees, chewable tablets, effervescent tablets, dispersible tablets, troches, melts, solutions, suspensions, emulsions and the like. The pharmaceutical preparations can be prepared by methods known in the related art.

The pharmaceutical preparation may be administered via any route, such as via infusion or bolus injection, or via administration route of absorption through the epithelium or mucocutaneous (e.g., oral mucosa or rectum, etc.). Administration can be systemic or local. Examples of preparations for oral administration include solid or liquid dosage forms, specifically, including tablets, pills, granules, powders, capsules, syrups, emulsions, suspensions and the like. The preparations can be prepared by methods known in the related art and include carriers, diluents or excipients conventionally used in the field of pharmaceutical preparations.

The present disclosure also provides use of the aforementioned solid dispersion, the pharmaceutical composition and the pharmaceutical preparation in the preparation of a medicament for the treatment and/or prevention of P2X3-associated diseases.

In an embodiment of the present disclosure, the P2X3-associated diseases are pain, respiratory system diseases or genitourinary system diseases.

In an embodiment of the present disclosure, the pain is chronic pain, acute pain, endometriosis-associated pain, neuropathic pain, back pain, cancer pain, inflammatory pain, postoperative pain, migraine or visceral pain;
and/or, the genitourinary system diseases are reduced bladder capacity, frequent micturition, urge incontinence, stress incontinence, bladder hyperreactivity, benign prostatic hyperplasia, prostatitis, detrusor hyperreflexia, pollakiuria, nocturia, urgency, overactive bladder, pelvic hypersensitivity, urethritis, pelvic pain syndrome, prostatodynia, or cystitis;
and/or, the respiratory system diseases are chronic obstructive pulmonary disease, pulmonary arterial hypertension, pulmonary fibrosis, asthma and obstructive sleep apnea, chronic cough, refractory chronic cough or acute cough.

The present disclosure also provides a method for the treatment and/or prevention of P2X3-associated diseases, which comprises administering an effective amount of the aforementioned solid dispersion, pharmaceutical composition or pharmaceutical preparation to a patient suffering from P2X3-associated diseases.

In an embodiment of the present disclosure, the P2X3-associated diseases are pain, respiratory system diseases or genitourinary system diseases.

In an embodiment of the present disclosure, the pain is chronic pain, acute pain, endometriosis-associated pain, neuropathic pain, back pain, cancer pain, inflammatory pain, postoperative pain, migraine or visceral pain;
and/or, the genitourinary system diseases are reduced bladder capacity, frequent micturition, urge incontinence, stress incontinence, bladder hyperreactivity, benign prostatic hyperplasia, prostatitis, detrusor hyperreflexia, pollakiuria, nocturia, urgency, overactive bladder, pelvic hypersensitivity, urethritis, pelvic pain syndrome, prostatodynia, or cystitis;
and/or, the respiratory system diseases are chronic obstructive pulmonary disease, pulmonary arterial hypertension, pulmonary fibrosis, asthma and obstructive sleep apnea, chronic cough, refractory chronic cough or acute cough.

### Terms and Definitions

Unless otherwise indicated, terms and definitions used in the present disclosure, including the specification and claims, are defined as follows.

Those skilled in the art will understand that, according to the usual practice in the related art, 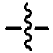 in the structural formula of the present disclosure is used to delineate a chemical bond, which is the point at which a moiety or substituent is connected to the core structure or the backbone structure.

The term "pharmaceutically acceptable" is referred for the compounds, materials, compositions and/or dosage forms, and means that they are suitable for use in contact with human and animal tissues without excess toxicity, irritation, allergic reactions or other problems or complications within the scope of reliable medical judgment, and are commensurate with a reasonable benefit/risk ratio.

The term "pharmaceutically acceptable salt" refers to salts of pharmaceutically acceptable non-toxic acids or bases, including salts of inorganic acids and bases, and salts of organic acids and bases.

The term "solvate" refers to the compound of the present disclosure or a salt thereof bonded with a stoichiometric or non-stoichiometric solvent through an intermolecular non-covalent force. When the solvent is water, the solvate is a hydrate.

The term "prodrug" refers to substances that can be converted, under physiological conditions or through solvolysis, into the compound of the present disclosure having biological activity. The prodrug of the present disclosure is prepared by modifying the functional groups in the compound, and the modification can be removed by conventional operations or removed in vivo, to obtain the parent compound. The prodrug includes a compound which is formed by connecting a hydroxyl group or amino group in the compound of the present disclosure to any group. When the prodrug of the compound of the present disclosure is administered to a mammal individual, the prodrug is dissociated to form a free hydroxyl or amino group.

The term "stereoisomer" refers to an isomer produced by a different spatial arrangement of atoms in the molecule, including cis and trans isomers, enantiomers, diastereomers, and conformers.

In accordance with the selection of raw materials and methods, the compound of the present disclosure may exist in the form of one of the possible isomers or a mixture thereof, for example, as a pure optical isomer, or as a mixture of isomers such as a racemic mixture and a diastereoisomeric mixture, depending on the number of asymmetric carbon atoms. When describing a compound with an optical activity, the prefixes D and L, or R and S are used to denote the absolute configuration of the molecule with respect to the chiral center (or multiple chiral centers) in the molecule. The prefixes D and L, or (+) and (-) are symbols used to specify a rotation of plane-polarized light caused by a compound, where (-) or L indicates that the compound is levorotatory, and the prefix (+) or D indicates that the compound is dextrorotatory. For a given chemical structure, these stereoisomers are identical except that these stereoisomers are mirror images of each other. Specific stereoisomers can be referred to as enantiomers, and a mixture of such isomers is generally called an enantiomeric mixture. A mixture of enantiomers in 50: 50 is called a racemic mixture or a racemate, which may occur when there is no stereoselectivity or stereospecificity in a chemical reaction or process. Numerous geometric isomers of olefins, C=N double bonds, etc. may also exist in the compounds described herein, and all such stable isomers shall be contemplated in the present disclosure. If the compounds described herein contain olefinic double bonds, such double bonds include both E-and Z-geometric isomers, unless otherwise specified. If the compound contains a disubstituted cycloalkyl, the substituent of the cycloalkyl may have a cis- or trans-conformation.

When the bond with a chiral carbon in the formula of the present disclosure is depicted in a straight line, it will be understood that the two configurations (R) and (S) of the chiral carbon and both the resulting enantiomerically pure compound and mixture are included in the scope defined by the general formula. The diagrammatic presentation of the racemate or enantiomerically pure compound herein is from Maehr, J. Chem. Ed. 1985, 62: 114-120. Unless otherwise specified, the wedge bond and the dashed bond are used to represent the absolute configuration of a stereocenter.

Optically active (R)-or (S)-isomers can be prepared using chiral synthons or chiral reagents, or resolved using conventional techniques. The compounds of the present disclosure containing asymmetrically substituted carbon atoms can be separated in an optically active form or in a racemic form. The resolution of a racemic mixture of a compound can be carried out with any of a variety of methods known in the art. Exemplary methods include fractional recrystallization using chiral resolving acids, which are optically active salt-forming organic acids. For example, the suitable resolving agents for fractional recrystallization are optically active acids, such as tartaric acid, diacetyl tartaric acid, dibenzoyl tartaric acid, mandelic acid, malic acid, lactic acid, or various optically active camphor sulfonic acids such as the *D* and *L* forms of β-camphor sulfonic acid. Other resolving agents suitable for fractional crystallization include α-methyl-benzylamine in a pure stereoisomeric form (for example, *S* and *R* forms or a diastereomerically pure form), 2-phenylglycinol, norephedrine, ephedrine, N-methylephedrine, cyclohexylethylamine, 1,2-diaminocyclohexane, etc. The resolution of the racemic mixture can also be carried out by elution on a column filled with an optically active resolving agent (for example, dinitrobenzoylphenylglycine). High performance liquid chromatography (HPLC) or supercritical fluid chromatography (SFC) can also be employed. The specific method, elution conditions, and the chromatographic columns can be selected by those skilled in the art according to the structures of the compounds and the experimental results. Further, pure optically active starting materials or reagents with known configurations can also be used to obtain any enantiomers or diastereomers of the compounds described in the present disclosure through stereo-organic synthesis.

The term "tautomer" refers to an isomer of a functional group resulting from a rapid movement of an atom between two positions in a molecule. The compound of the present disclosure may exhibit tautomerism. Tautomeric compounds can be present in two or more mutually convertible species. The prototropic tautomer is resulted from a migration of covalently bonded hydrogen atoms between two atoms. The tautomer generally exists in an equilibrium form. When trying to separate a single tautomer, a mixture is usually produced, the physical and chemical properties of which are consistent with the mixture of compounds. The position of equilibrium depends on the intramolecularly chemical properties. For example, for many aliphatic aldehydes and ketones, such as acetaldehyde, the ketonic type is dominant; and for phenols, the enol type is dominant. All tautomeric forms of the compounds are included in the present disclosure.

The compound of the present disclosure may contain atomic isotopes in an unnatural ratio on one or more of the atoms constituting the compound. For example, the compound may be labeled with a radioisotope, such as deuterium (²H), tritium (³H), iodine-125 (¹²⁵I), or C-14 (¹⁴C). The transformation of all isotopic compositions of the compounds of the present disclosure, whether radioactive or not, are included within the scope of the present disclosure.

With respect to a drug or pharmacologically active agent, the term "effective amount" or "therapeutically effective amount" refers to a non-toxic but sufficient amount of the drug or agent to achieve the desired effects. For oral dosage forms in the present disclosure, an "effective amount" of one active substance in a composition refers to an amount required to achieve the desired effects when used in combination with another active substance in the composition. The determination of the effective amount varies from person to person, depending on the age and general condition of the subject, as well as the specific active substance. The appropriate effective amount in individual cases can be determined by those skilled in the art based on routine experiments.

The terms "active component", "therapeutic agent", "active substance", or "active agent" refer to a chemical entity that is effective in treating the target disorder, disease, or condition.

The term "substituted" means that any one or more hydrogen atoms on a specific atom are replaced by a substituent, including deuterium and hydrogen variants, as long as the valence of the specific atom is normal and the substituted compound is stable. When the substituent is keto (i.e., =O), it means that two hydrogen atoms are substituted. Ketone substitution does not occur on aromatic groups. The term "optionally substituted" means that it is substituted or unsubstituted. Unless otherwise specified, the type and number of substituents may be arbitrary on a chemically achievable basis.

The term "C₁-C₆ alkyl" refers to a linear or branched, saturated, monovalent hydrocarbon group having 1, 2, 3, 4, 5, or 6 carbon atoms. Said alkyl is, for example, methyl, ethyl, propyl, butyl, pentyl, hexyl, isopropyl, isobutyl, sec-butyl, tert-butyl, isoamyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2, 2-dimethylbutyl, 1, 1-dimethylbutyl, 2, 3-dimethylbutyl, 1, 3-dimethylbutyl, 1, 2-dimethylbutyl, etc., or an isomer thereof. Specifically, said group has 1, 2, 3, or 4 carbon atoms ("C₁-C₄ alkyl"), e.g., methyl, ethyl, n-propyl, isopropyl, n-butyl, isobutyl, sec-butyl, or tert-butyl.

The term "C₁-C₃ alkoxy" should be understood as -O-(C₁-C₃ alkyl), where "C₁-C₃ alkyl" is defined as above.

The term "deuterated C₁-C₆ alkyl" should be understood as C₁-C₆ alkyl in which one or more hydrogen atoms are substituted by deuterium, where "C₁-C₆ alkyl" is defined as above.

The term "C₃-C₇ cycloalkyl" should be understood as a saturated monovalent monocyclic or bicyclic hydrocarbon ring having 3 to 7 carbon atoms, including fused or bridged polycyclic ring systems. Similarly, "C₃-C₆ cycloalkyl" should be understood as a saturated monovalent monocyclic or bicyclic hydrocarbon ring as defined above having 3 to 6 carbon atoms. Such examples include, but are not limited to, cyclopropyl, cyclobutyl, cyclopentyl, and cyclohexyl.

The term "alkylene" should be understood as a saturated divalent hydrocarbyl group obtained by removing two hydrogen atoms from a saturated straight or branched hydrocarbon. Unless otherwise specified, alkylene contain 1 to 10 carbon atoms. In some embodiments, alkylene contains 1 to 6 carbon atoms; in some other embodiments, alkylene contains 1 to 4 carbon atoms; in still some other embodiments, alkylene contains 1 to 2 carbon atoms. Such examples include, but are not limited to, methylene (-CH₂-), ethylene (-CH₂CH₂-), n-propylene (-CH₂CH₂CH₂-), isopropylidene (-CH(CH₃)CH₂-), etc.

The term "heterocycloalkyl" should be understood as a saturated monovalent monocyclic hydrocarbon ring having the indicated number of ring atoms, in which one, two or three ring atoms of the hydrocarbon ring are independently replaced by one, two, or three heteroatoms or heteroatom-containing groups, which are independently selected from O, S, S(=O), S(=O)₂, or N. The term "4-7-membered heterocycloalkyl" should be understood as a saturated monovalent monocyclic "heterocycloalkyl" ring as defined above containing 4, 5, 6, or 7 ring atoms. Similarly, the term "4-6-membered heterocycloalkyl" should be understood as a saturated monovalent monocyclic "heterocycloalkyl" ring as defined above containing 4, 5, or 6 ring atoms.

The term "6-12-membered heterobicycloalkyl" should be understood as a saturated monovalent bicyclic hydrocarbyl group containing 5, 6, 7, 8, 9, or 10 carbon atoms and one, two, or three heteroatoms or heteroatom-containing groups independently selected from O, S, S (=O), S(=O)₂ , or N, in which two rings share one or two common ring atoms, provided that the total number of ring atoms is not greater than 12.

The term "5-8-membered aryl" should be understood as a monovalent aromatic or partially aromatic monocyclic, bicyclic, or tricyclic hydrocarbon ring having 5 to 8 carbon atoms, especially a ring having 6 carbon atoms ("C₆ aryl"), such as phenyl. When the 5-8-membered aryl is substituted, it may be mono-or poly-substituted, and the substitution site is not limited, for example, it may be ortho-, para-, or meta-substituted.

The term "5-10-membered heteroaryl" should be understood as a monovalent monocyclic, bicyclic, or tricyclic aromatic ring group having 5 to 10 ring atoms (especially, 5 or 6 carbon atoms) and 1 to 5 heteroatoms (preferably 1 to 3 heteroatoms) independently selected from N, O, or S. In addition, in each case, it may be benzo-fused group. Particularly, heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl, etc.; or pyridyl, pyridazinyl, pyrimidinyl, pyrazinyl, triazinyl, etc.; cinnolinyl, phthalazinyl, quinazolinyl, quinoxalinyl, naphthyridinyl, pteridinyl, carbazolyl, acridinyl, phenazinyl, phenothiazinyl, phenoxazinyl, etc.

The term "halogen substituent" or "halogen" is fluorine, chlorine, bromine, or iodine.

In addition, it should be noted that, unless clearly stated otherwise, the description involving "...independently" in the present disclosure should be understood in a broad sense, meaning that the described individuals are independent of each other and may be independently the same or different specific groups. In more detail, the description involving "...independently" can either mean that among different groups, the specific options expressed by the same symbols do not affect each other, or it can mean that in the same group, the specific options expressed by the same symbols do not affect each other.

### Beneficial Effects

The present disclosure provides the solid dispersion containing the P2X3 receptor antagonist compound represented by Formula I, which can significantly improve the solubility of the compound represented by Formula I, and greatly improve the dissolution rate and bioavailability of the drug, ensuring therapeutic effect of the drug. In addition, the solid dispersion of the present disclosure has good stability. During the storage process, the solid dispersion does not experience crystal form tranformation, which improves the safety of the clinical use of the drug.

The present disclosure has carried out extensive solid dispersion screening for the compound of Formula A, including different types of solid dispersion carriers and drug-carrier ratios, which obtained the solid dispersion with a relatively high drug-carrier ratio and excellent drug stability. When administered to canine at a dose of 20 mg/kg, the solid dispersion achieves favorable exposure, and can maintain a supersaturated concentration for a relatively long period of time, with stable and sustained in vivo absorption remaining observable after 4 hours.

Pharmacokinetic study in rat, mouce and canine show that, Compounds I-27, I-28, I-29 and I-30 have comparable pharmacokinetic properties.

### BRIEF DESCRIPTION OF THE DRAWINGS

Figure 1: XRPD Pattern of the API Compound.
Figure 2: TGA & DSC Curves of the API Compound.
Figure 3: Dynamic dissolution curves of amorphous films of API with HPMC-AS-LF at different ratios.
Figure 4: Dynamic dissolution curves of amorphous films of API with Eudragit L100-55 at different ratios.
Figure 5: Dynamic dissolution curves of amorphous films of API with HPMC-E5 at different ratios.
Figure 6: Dynamic dissolution curves of amorphous films of API with PVPVA64 at different ratios.
Figure 7: Dynamic dissolution curves of amorphous films of API with Soluplus at different ratios.
Figure 8: Solid-State XRPD patterns of preparation F25 for 7-day physical stability under various conditions.
Figure 9-1: Solid-State XRPD patterns of preparation F25 for 18-day physical stability at 40 °C and 60% RH.
Figure 9-2: Solid-State XRPD patterns of preparation F25 for 18-day physical stability at 40 °C and 75% RH.
Figure 9-3: Solid-State XRPD patterns of preparation F25 for 18-day physical stability at 60 °C and 30% RH.
Figure 10: Solid-State XRPD patterns of preparation F26 for 7-day physical stability under various conditions.
Figure 11: Solid-State XRPD patterns of preparation F26 for 18-day physical stability under various conditions.

### DETAILED DESCRIPTION

The technical solutions of the present disclosure will be further described in detail with reference to the following specific examples. It should be understood that the following examples are merely exemplary illustration and explanation of the present disclosure and should not be construed as limitations to the protection scope of the present disclosure. All techniques implemented based on the contents described above of the present disclosure are encompassed within the protection scope of the present disclosure.

Unless otherwise stated, the starting materials and reagents used in the following examples are all commercially available products or can be prepared by using known methods.

The compounds of the present application and preparation thereof are referred to WO2022068930A1 and WO2023185931A1.

The term "pharmaceutically acceptable" refers to excipients that, within the scope of sound medical judgment, are suitable for use in contact with human and animal tissues, without causing excessive toxicity, irritation, allergic reactions or other problems or complications, and have a reasonable benefit/risk ratio.

### Abbreviation List

**Table 1-1 Abbreviations and corresponding full names thereof in the invention**

| **Abbreviation** | **Full name** |
|---|---|
| API | Active Ingredient (Compound of Formula A, i.e., Compound of Formula I-27) |
| SD | Solid Dispersion |
| SDD | Spray-Dried Solid Dispersion |
| HPMC-E5 | Hypromellose E5 |
| HPMC-AS-LF | Hypromellose Acetate Succinate LF |
| Eudragit-L100-55 | Eudragit L100-55 |

| Soluplus | Polyvinylcaprolactam-Polyvinyl Acetate-Polyethylene Glycol Graft Copolymer |
|---|---|
| PVP VA64 | Copovidone VA64 |
| DCM | Dichloromethane |
| SGF | Simulated Gastric Fluid |
| FaSSIF | Simulated Fasted Intestinal Fluid |
| HPLC | High-Performance Liquid Chromatograph |
| MeOH | Methanol |
| XRD | X-ray Powder Diffractometer |
| RH | Relative Humidity |
| XRPD | X-ray Powder Diffraction |
| TGA | Thermogravimetric Analysis |
| DSC | Differential Scanning Calorimetry |
| DVS | Dynamic Vapor Sorption |

### Instrument and Detection Method

### 1. Experimental Instrument

The models and manufacturers of the main instruments used in the present disclosure are shown in Table 2-1:

**Table 2-1 Main Instruments Used in the Experiment**

| **Name** | **Model** | **Manufacturer** |
|---|---|---|
| Electronic Balance | XS5205DU | Mettler Toledo |
| X-ray Diffractometer | Aeris Research | Malvern Panalytial |
| Differential Scanning Calorimetry | DSC250 | TA |
| Thermogravimetric Analyzer | TGA550 | TA |
| Dynamic Vapor Sorption Analyzer | Intrinsic Plus | SMS |
| Polarizing Microscope | Axio ObserverVett. A1 | ZEISS |
| HPLC | 1260 Infinity II | Aiglient |
| Spray Dryer | ADL311S (GAS410 Organic Solvent Recovery Unit) | Yamato |
| Vacuum Drying Oven | PZG-6050D | Shanghai Senxin Experimental Instrument Co., Ltd. |
| 96-Channel Pipette | Liquidator 96 | Mettler Toledo |
| Constant Temperature Shaker | ZQTY-70N | Shanghai Zhichu Instrument Co., Ltd. |

### 2. X-ray Powder Diffraction (XRPD)

The relevant parameters of the X-ray Powder Diffraction (XRPD) instrument are shown in Table 2-2:

**Table 2-2 Relevant Parameters of the XRPD Instrument**

| Instrument | Malvern Panalytical Aeris |
|---|---|
| X-ray Wavelength | Cu, Kα:1.5419Å |
| X-ray Tube Setting | 40 kV, 15 mA |
| Scanning Mode | 1D |
| Scanning Rang (2θ) | 3.5°-40° |
| Scanning step (2θ) | 0.0027166° |
| Scanning Rate (2θ) | 10°/min |

### 3. Thermogravimetric Analysis (TGA)

The relevant parameters of the TGA instrument are shown in Table 2-3:

**Table 2-3 Relevant Parameters of the TGA Instrument**

| Experimental Instrument | TA, TGA500 |
|---|---|
| Test Method | Linear Temperature Ramp |
| Temperature Range | 25-300.00°C |
| Heating Rate | 10 °C /min |
| Purge Gas | N₂ (>99.999%) |
| Sample Pan Type | Aluminum open sample pan, no lid |

### 4. Differential Scanning Calorimeter (DSC)

The relevant parameters of the DSC instrument are shown in Table 2-4.

**Table 2-4 Relevant Parameters of the DSC Instrument**

| Experimental Instrument | TA, DSC250 |
|---|---|
| Test Method | Linear Temperature Ramp |
| Temperature Range | RT -300.00 °C |
| Heating Rate | 10 °C /min |
| Purge Gas | N₂ (>99.999%) |
| Sample Pan Type | Aluminum sample pan with perforated lid |

### 5. Dynamic Vapor Sorption (DVS)

DVS tests were performed at 25°C with a relative humidity range of 0-90% RH and a dm/dt value of 0.002%/min to record the dynamic water vapor sorption curves. The relevant parameters of the test instrument are shown in Table 2-5.

**Table 2-5 Test Methods of the DVS Instrument**

| **Experimental Instrument** | **DVS, Intrinsic Plus** |
|---|---|
| Humidity Range | 50% RH-0% RH-90% RH-0% RH-50% RH |
| Humidity Gradient | 10% RH per step |
| Test Temperature | 25 °C |
| step Switching Method | dm/dt :0.002% |
| Cycle Number | 1 |
| Gas Flow Rate | N₂ 200 mL/min |

### 6. High Performance Liquid Chromatography (HPLC) Analytical Method

The HPLC test methods and relevant parameters are shown in Table 2-6.

**Table 2-6 HPLC Analytical Method (Physiological Medium-Related Dissolution Determination)**

| Chromatographic Column | Thermo Acclaim^{™} 120 C18 4.6 × 100 mm 5 µm |
|---|---|
| Column Temperature | 35°C |
| Wavelength | 210 nm |
| Flow Rate | 1 mL/min |
| Injection Volume | 10 µL |
| Mobile Phase | Mobile Phase A: 0.1% Perchloric acid solution |
| | Mobile Phase B: Acetonitrile |
| Elution Gradient | Isocratic Elution, Mobile Phase A - Mobile Phase B (40:60, v%:v%) |
| Solvent | 50% Acetonitrile Solution |
| Reference Standard Solution | 0.05 mg/mL (Light-shielding) |

### Reagents, Excipients and Manufacturers

**Table 3-1 Reagents, Excipients and Manufacturers used in the Present disclosure**

| **Reagent or Excipient** | **Model and Manufacturer** |
|---|---|
| HPMC-E5 | MedoShine^{™}, Colorcon |
| HPMC-AS-LF | AQOAT^{®}, Shin-Etsu |
| Eudragit-L100-55 | Eudragit^{®}, EVONIK |
| Soluplus | Soluplus^{®}, BASF |
| PVP VA64 | Kollidon^{®}, BASF |
| FaSSIF | biorelevant |
| FaSSGF | biorelevant |

### Preparation of API

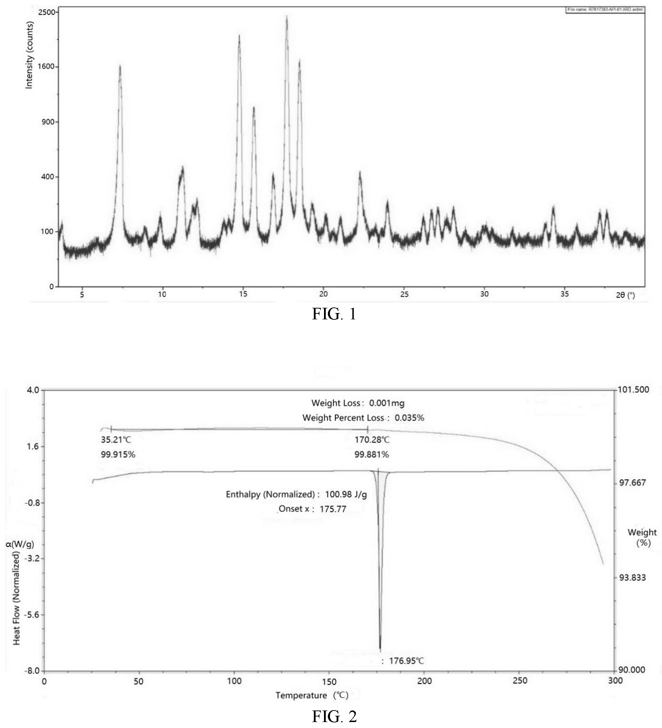

The compound of Formula A (I-27) as API was prepared according to the methods described in WO2022068930A1 and WO2023185931A1. The obtained product was characterized by XRPD (Figure 1), DSC (Figure 2) and TGA (Figure 2), and was confirmed to be the free base Crystal Form A disclosed in WO2023185931A1.

### Example 1 Screening of Excipients for Solid Dispersion

### 1. Screening Study on Carriers of Amorphous Solid Dispersions

**Table 4-1 Screening Study on Carriers of Amorphous Solid Dispersions**

| **Investigation Content** | **carrier** | 96-well plate: Row arrangement | Formulation F1~F20 | | | | | | | | | | | | | |
|---|---|---|---|---|---|---|---|---|---|---|---|---|---|---|---|---|
| | | | 1 | 2 | 3 | 4 | | 5 | 6 | 7 | 8 | | 9 | 10 | 11 | 12 |
| | | | **API: carrier** | | | | | | | | | | | | | **API** |
| | | | **1:1** | | | | **1:2** | | | | | **1:3** | | | | |
| | HPMC-AS-LF | A | F1 | F1 | F1 | F1 | | F2 | F2 | F2 | F2 | | F3 | F3 | F3 | F16 |
| **SDD without sedimentation inhibitor addition** | Eudragit L100-55 | B | F4 | F4 | F4 | F4 | | F5 | F5 | F5 | F5 | | F6 | F6 | F6 | F17 |
| | HPMC-E5 | C | F7 | F7 | F7 | F7 | | F8 | F8 | F8 | F8 | | F9 | F9 | F9 | F18 |
| | PVP VA64 | D | F10 | F10 | F10 | F10 | | F11 | F11 | F11 | F11 | | F12 | F12 | F12 | F19 |
| | Soluplus | E | F13 | F13 | F13 | F13 | | F14 | F14 | F14 | F14 | | F15 | F15 | F15 | F20 |

The compound reprented by Formula A exhibits high solubility in a dichloromethane/methanol system (volume ratio = 1:1). This solvent system was used to prepare an amorphous solid dispersion thin film, and the experimental procedures are as follows:
1) Thin films of the amorphous solid dispersions were prepared in 96-well plates according to the mass ratios listed in Table 4-1. A total of ten identical 96-well plates were prepared, with each well containing 400 µg of API (generally 10 times the maximum saturated solubility in physiological solutions).
2) A total of ten 96-well plates were prepared via rapid solvent evaporation method, among which 8 plates were used for physiologically relevant dissolution tests, 1 plate for physical stability evaluation, and 1 plate served as the excipient control. The detailed information is shown in Table 4-1.
3) Eight 96-well plates loaded with amorphous solid dispersion thin films were subjected to physiologically relevant dissolution tests. Sampling (96-channel), filtration (96-channel) and dilution (96-channel) were performed at designated time points: 5 min and 10 min in simulated gastric fluid (SGF); medium replacement in concentrated fasted state simulated intestinal fluid (FaSSIF) at 15 min, followed by sampling at 20 min, 30 min, 45 min, 60 min, 90 min and 120 min.
4) The post-dissolution samples above were analyzed by HPLC to reconstruct the 96-channel physiologically relevant dissolution profile.
5) The plates for physical stability evaluation and blank excipient plates were subjected to high-throughput cross-polarized light imaging at T0 and Day 7 (40°C / 75% RH), to evaluate the physical stability of the formulations.
6) By comparing the differences of the formulations, carriers and sedimentation inhibitors exhibiting favorable dynamic dissolution behaviors were selected for spray drying to prepare solid dispersion (SDD).

### 2. Dissolution Behavior of Amorphous Thin Films with Different Carrier Types and Ratios

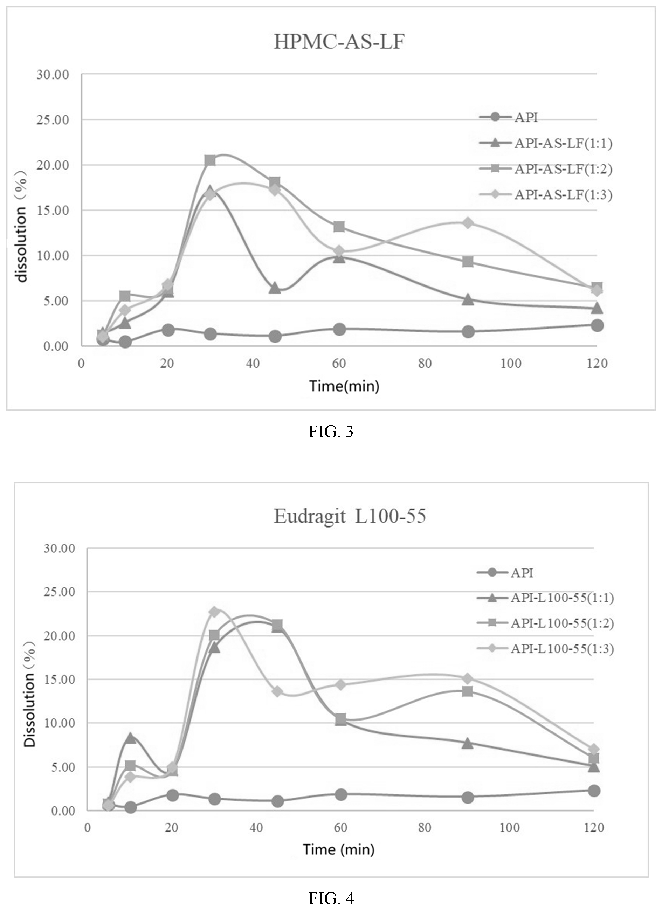

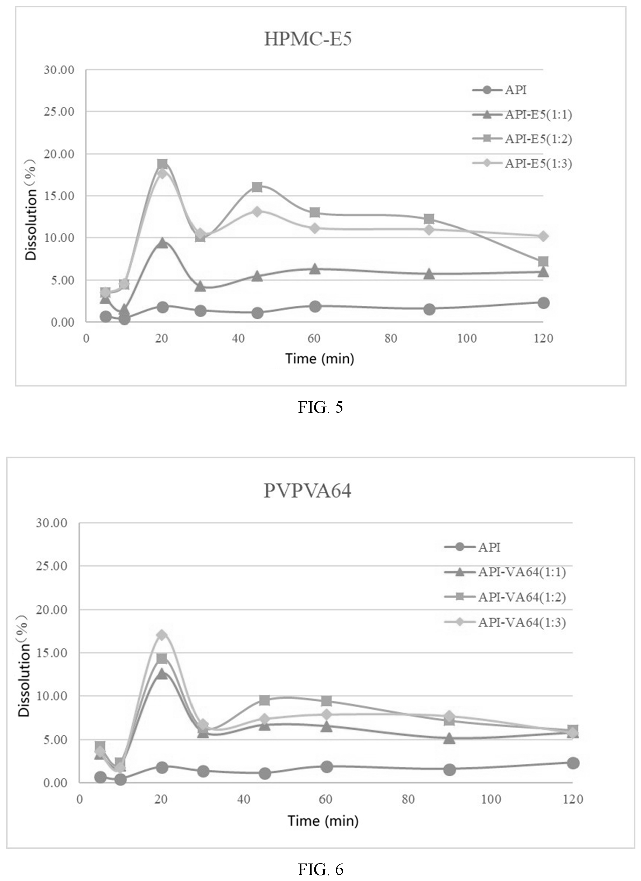

The prepared amorphous solid dispersion thin films containing API with different carrier types and ratios were subjected to dynamic solubility tests in physiologically relevant media. The results were compared with the dynamic solubility data of amorphous API thin films, and dynamic dissolution curves were plotted. The experimental results are shown in Table 4-4 to Table 4-8 and Figure 3 to Figure 7.

The test method is as follows:

**Table 4-2 Dynamic Solubility Test Method in Physiologically Relevant Media**

| | |
|---|---|
| Dissolution Apparatus | FOCS Dissolution Apparatus |
| Dissolution Method | Paddle Method |
| Rotation Speed | 75 rpm |
| Medium | SGF medium 15min, SGF+FaSSIF medium 105min |
| Medium Volume | 300 mL, 300mL+600mL |
| Medium Temperature | 37°C±0.5°C |
| Sampling Time | 5min, 10min, 20min, 30min, 45min, 60min, 90min, 120min |

### Preparation of physiologically relevant media:

The preparation methods of SGF and concentrated FaSSIF solution are shown in the table below. The pH of concentrated FaSSIF solution was pre-adjusted with 1.0 mol/L aqueous sodium hydroxide solution, so that the pH value could reach 6.5 after SGF and concentrated FaSSIF were mixed uniformly at a volume ratio of 1:2.

**Table 4-3 Preparation Method of SGF and Concentrated FaSSIF Solution**

| Material Name | Sample Weighing Amount (g/200ml) | |
|---|---|---|
| | SGF | Concentrated FaSSIF |
| Sodium Chloride | 0.4 | 1.6558 |
| Sodium Hydroxide | / | 0.126 |
| Sodium dihydrogen phosphate | / | 1.0314 |
| FaSSIF or FaSSGF | 0.012 | 0.666 |
| pH | 1.6 | Q.S* |

| | | |
|---|---|---|
| * Adjustment shall be performed according to the pH requirement of the mixed solution. | | |

**Table 4-4 Dynamic Dissolution Results of Amorphous Thin Films Composed of API and HPMC-AS-LF at Different Ratios**

| | **Time (min)** | | | | | | | |
|---|---|---|---|---|---|---|---|---|
| | **5** | **10** | **20** | **30** | **45** | **60** | **90** | **120** |
| API | 0.70 | 0.44 | 1.81 | 1.35 | 1.11 | 1.85 | 1.56 | 2.31 |
| AS LF-1:1 | 1.43 | 2.59 | 5.99 | 17.10 | 6.40 | 9.80 | 5.18 | 4.15 |
| AS LF-1:2 | 1.13 | 5.47 | 6.36 | 20.48 | 18.07 | 13.16 | 9.27 | 6.38 |
| AS LF-1:3 | 0.98 | 3.90 | 6.79 | 16.65 | 17.21 | 10.52 | 13.54 | 6.06 |

| | | | | | | | | |
|---|---|---|---|---|---|---|---|---|
| In Table 4-4, AS LF-1:1 represents API:HPMC-AS-LF=1:1. AS LF-1:2 represents API:HPMC-AS-LF=1:2, and AS LF-1:3 represents API:HPMC-AS-LF=1:3. | | | | | | | | |

**Table 4-5 Dynamic Dissolution Results of Amorphous Thin Films Composed of API and Eudragit L100-55 at Different Ratios**

| | **Time (min)** | | | | | | | |
|---|---|---|---|---|---|---|---|---|
| | **5** | **10** | **20** | **30** | **45** | **60** | **90** | **120** |
| API | 0.70 | 0.44 | 1.81 | 1.35 | 1.11 | 1.85 | 1.56 | 2.31 |
| L100-55-1:1 | 0.94 | 8.34 | 4.58 | 18.68 | 20.97 | 10.36 | 7.75 | 5.08 |
| L100-55-1:2 | 0.73 | 5.17 | 4.51 | 20.03 | 21.29 | 10.58 | 13.63 | 6.03 |
| L100-55-1:3 | 0.58 | 3.85 | 4.95 | 22.73 | 13.63 | 14.38 | 15.11 | 7.01 |

| | | | | | | | | |
|---|---|---|---|---|---|---|---|---|
| In Table 4-5, L100-55-1:1 represents API: Eudragit-L100-55=1:1, L100-55-1:2 represents API: Eudragit-L100-55=1:2, and L100-55-1:3 represents API: Eudragit-L100-55=1:3. | | | | | | | | |

**Table 4-6 Dynamic Dissolution Results of Amorphous Thin Films Composed of API and HPMC-E5 with Different Ratios**

| | **Time (min)** | | | | | | | |
|---|---|---|---|---|---|---|---|---|
| | **5** | **10** | **20** | **30** | **45** | **60** | **90** | **120** |
| API | 0.70 | 0.44 | 1.81 | 1.35 | 1.11 | 1.85 | 1.56 | 2.31 |
| E5-1:1 | 2.86 | 1.57 | 9.46 | 4.28 | 5.48 | 6.30 | 5.75 | 5.98 |
| E5-1:2 | 3.47 | 4.44 | 18.80 | 10.13 | 16.06 | 12.99 | 12.22 | 7.19 |
| E5-1:3 | 3.48 | 4.58 | 17.63 | 10.58 | 13.11 | 11.18 | 10.99 | 10.22 |

| | | | | | | | | |
|---|---|---|---|---|---|---|---|---|
| In Table 4-6, E5-1:1 represents API: HPMC E5=1:1, E5-1:2 represents API: HPMC E5=1:2, and E5-1:3 represents API: HPMC E5=1:3. | | | | | | | | |

**Table 4-7 Dynamic Dissolution Results of Amorphous Thin Films Composed of API and PVP VA64 at Different Ratios**

| | **Time (min)** | | | | | | | |
|---|---|---|---|---|---|---|---|---|
| | **5** | **10** | **20** | **30** | **45** | **60** | **90** | **120** |
| API | 0.70 | 0.44 | 1.81 | 1.35 | 1.11 | 1.85 | 1.56 | 2.31 |
| VA64-1:1 | 3.38 | 1.97 | 12.59 | 5.85 | 6.67 | 6.54 | 5.15 | 5.78 |
| VA64-1:2 | 4.15 | 2.31 | 14.29 | 6.30 | 9.53 | 9.42 | 7.16 | 6.05 |
| VA64-1:3 | 3.58 | 1.74 | 17.03 | 6.75 | 7.39 | 7.88 | 7.69 | 5.78 |

| | | | | | | | | |
|---|---|---|---|---|---|---|---|---|
| In Table 4-7, VA64-1:1 represents API: PVP VA64=1:1, VA64-1:2 represents API: PVP VA64=1:2, and VA64-1:3 represents API: PVP VA64=1:3. | | | | | | | | |

**Table 4-8 Dynamic Dissolution Results of Amorphous Thin Films Composed of API and Soluplus at Different Ratios**

| | **Time (min)** | | | | | | | |
|---|---|---|---|---|---|---|---|---|
| | **5** | **10** | **20** | **30** | **45** | **60** | **90** | **120** |
| API | 0.70 | 0.44 | 1.81 | 1.35 | 1.11 | 1.85 | 1.56 | 2.31 |
| Soluplus-1:1 | 2.03 | 1.31 | 7.55 | 9.45 | 5.96 | 7.95 | 6.54 | 6.64 |
| Soluplus-1:2 | 0.36 | 1.17 | 1.03 | 0.90 | 0.87 | 1.01 | 2.76 | 3.01 |
| Soluplus-1:3 | 1.69 | 1.71 | 6.81 | 12.15 | 5.10 | 9.11 | 7.20 | 9.21 |

| | | | | | | | | |
|---|---|---|---|---|---|---|---|---|
| In Table 4-8, Soluplus-1:1 represents API: Soluplus=1:1, Soluplus-1:2 represents API: Soluplus=1:2, and Soluplus-1:3 represents API: Soluplus=1:3. | | | | | | | | |

### 3. Summary of Screening Study on Amorphous Solid Dispersion

Based on the above experimental results (Table 4-4 to Table 4-8 and Figure 3-7), compared with the pure API control group, the dissolution performance of SDDs prepared with different carriers (HPMC-E5, HPMC-AS-LF, Eudragit L100-55, PVP VA64 and Soluplus) was enhanced at different levels. Among them, SDDs formulated with the HPMC-E5, HPMC-AS-LF and Eudragit L100-55 exhibited the most significant dissolution enhancement.

### Example 2: Preparation of SDDs by Spray Drying and Investigation of Dissolution Profiles

Based on the above screening results, spray drying was used to prepare SDD formulations with carriers including the HPMC-E5, HPMC-AS-LF and Eudragit L100-55. Acetone was used as the solvent for the preparation of solid dispersions. The procedures are as follows:
(1) The carrier was slowly added into the solvent and continuously stirred for no less than 60 minutes until completely dissolved to form a clear solution.
(2) The compound was slowly added to the above solution, followed by continuous stirring for no less than 120 minutes until completely dissolved to form a clear solution.
(3) The resulting solution was pressure-filtered through a precision filter with a pore size of 5 µm into a transfer tank of appropriate volume.
(4) The pressure-filtered solution was subjected to spray drying with the following parameters: feed frequency of 20 Hz, atomization pressure of 0.2 MPa, inlet air temperature controlled at approximately 85-110°C, and outlet air temperature ranging from 40-70°C.
(5) The spray-dried solid dispersions were transferred to a vacuum drying oven for vacuum drying to obtain dried solid dispersions.

Finally, the solid dispersions of different formulations were subjected to physiologically relevant dissolution tests.

**Table 5-1 Summary of Formulation Information for F21~F28**

| **Formulation** | **F21** | **F22** | **F23** | **F24** | **F25** | **F26** | **F27** | **F28** |
|---|---|---|---|---|---|---|---|---|
| API | 50% | 33.3% | 25% | 50% | 33.3% | 25% | 33.3% | 25% |
| HPMC-E5 | 50% | 66.7% | 75% | / | / | / | / | / |
| HPMCAS-LF | / | / | / | 50% | 66.7% | 75% | / | / |
| Eudragit-L100-55 | / | / | / | / | / | / | 66.7% | 75% |
| Total | 100% | 100% | 100% | 100% | 100% | 100% | 100% | 100% |

The dissolution results are presented in Table 5-2 and Table 5-3.

**Table 5-2 Study on Dissolution Profiles of API : HPMC-E5 = 1:1, 1:2 and 1:3**

| **Time point** | **Sample** | **API:HPMC-E5=1:1 (n=3)** | | **API:HPMC-E5=1:2 (n=3)** | | **API:HPMC-E5=1:3 (n=3)** | |
|---|---|---|---|---|---|---|---|
| | **Formulation** | **F21** | | **F22** | | **F23** | |
| | **media** | **Dissolution %** | **RSD%** | **Dissolution %** | **RSD%** | **Dissolution %** | **RSD%** |
| 5min | 300mL SGF | 1.1 | 21.0 | 8.4 | 31.3 | 1.5 | 85.8 |
| 10min | | 3.1 | 41.5 | 8.0 | 18.2 | 3.7 | 34.8 |
| 15min | | 4.3 | 27.6 | 7.3 | 18.4 | 5.2 | 43.8 |
| 20min | 300mL SGF+600 mL FaSSIF | 2.9 | 25.9 | 8.4 | 15.5 | 9.4 | 8.0 |
| 30min | | 3.8 | 1.6 | 8.4 | 7.7 | 11.0 | 21.9 |
| 45min | | 4.6 | 16.4 | 9.7 | 7.8 | 12.3 | 20.0 |
| 60min | | 5.8 | 12.0 | 11.7 | 15.2 | 15.6 | 12.2 |
| 90min | | 7.8 | 9.7 | 12.2 | 6.5 | 21.3 | 8.9 |
| 120min | | 9.1 | 8.0 | 15.1 | 8.3 | 23.8 | 3.1 |

**Table 5-3 Dissolution Study on HPMC-AS-LF and Eudragit as SDD Carriers**

| **Time point** | **Sample** | **API:HPMC-AS-LF=1:1 (n=3)** | | **API:HPMC-AS-LF=1:2 (n=3)** | | **API:HPMC-AS-LF=1:3 (n=3)** | | **API:Eudragit L100-50=1:2 (n=3)** | | **API:Eudragit L100-50=1:3 (n=3)** | |
|---|---|---|---|---|---|---|---|---|---|---|---|
| | **Formulation** | **F24** | | **F25** | | **F26** | | **F27** | | **F28** | |
| | media | Dissolution % | RSD % | Dissolution % | RSD % | Dissolution % | RSD % | Dissolution % | RSD % | Dissolution % | RSD % |
| 5min | 300mL SGF | 2.9 | 8.0 | 12.3 | 12.4 | 13.9 | 13.0 | 4.0 | 10.2 | 4.7 | 17.9 |
| 10min | | 3.4 | 8.5 | 13.9 | 14.5 | 15.7 | 11.8 | 4.1 | 8.4 | 5.8 | 16.2 |
| 15min | | 3.8 | 6.1 | 15.2 | 14.2 | 17.1 | 10.9 | 4.9 | 5.3 | 6.8 | 12.6 |
| 20min | 300mL SGF+600 mL FaSSIF | 6.2 | 0.0 | 32.3 | 23.9 | 45.6 | 10.2 | 19.4 | 4.1 | 32.3 | 7.9 |
| 30min | | 7.4 | 0.0 | 35.3 | 23.1 | 63.0 | 11.3 | 27.9 | 4.3 | 42.9 | 4.5 |
| 45min | | 7.8 | 8.9 | 36.3 | 21.4 | 71.0 | 6.3 | 30.5 | 3.2 | 43.1 | 1.6 |
| 60min | | 7.1 | 25.9 | 36.0 | 20.9 | 71.8 | 5.3 | 30.6 | 1.5 | 38.1 | 3.1 |
| 90min | | 9.1 | 7.7 | 36.6 | 18.8 | 68.9 | 3.0 | 30.8 | 1.1 | 33.1 | 2.0 |
| 120min | | 10.4 | 6.7 | 36.8 | 13.1 | 64.1 | 1.3 | 28.6 | 0 | 31.3 | 0.2 |

Based on the dissolution test results of SDDs prepared with three different carriers (HPMC-E5, HPMC-AS-LF and Eudragit L100-55) in physiologically relevant media:
For the same carrier, the dissolution of SDD increased with the rise in carrier ratio, API: HPMC-AS-LF = 1:3 (dissolution rate 64.1%) > 1:2 (dissolution rate 36.8%) > 1:1 (dissolution rate 10.4%); API: HPMC-E5 = 1:3 (dissolution rate 23.8%) > 1:2 (dissolution rate 15.1%) > 1:1 (dissolution rate 9.1%); API: Eudragit L100-55 = 1:3 (dissolution rate 31.3%) > 1:2 (dissolution rate 28.6%). It indicated that the formulation of API: HPMC-AS-LF = 1:3 exhibited the best dissolution behavior, with a 60 min cumulative dissolution of 72%, followed by the API: HPMC-AS-LF = 1:2 group.

### Example 3: Physical Stability Study

Experimental procedures for physical stability study: Approximately 10.0 mg of the sample was weighed and placed into a 1.5mL HPLC vial. The samples were stored under different temperature and humidity conditions. At multiple time points in 3 days, 7 days and 18 days, samples were collected and characterized by XRD.

The results of the stability study are shown in Table 6-1, Table 6-2 and Table 6-3.

**Table 6-1 Physical Stability Results of Formulation API: HPMC-AS-LF (1:1)**

| **Formulation** | **Condition** | **3 days** | | **7 days** | | **18 days** | |
|---|---|---|---|---|---|---|---|
| | | **Appearance** | **Solid-state property** | **Appearance** | **Solid-state property** | **Appearance** | **Solid-state property** |
| F24 | 40°C, RH 60% | white powder | amorphous form | white powder | amorphous form | white powder | amorphous form |
| | 40°C, RH 75% | white powder (caking) | amorphous form | white hard lumps | crystal transformation | // | // |
| | 60°C, RH 30% | white powder | amorphous form | white powder | amorphous form | white powder | crystal transformation |
| | 60°C, RH 75% | white powder (caking) | crystal transformation | pale yellow hard particle | // | pale yellow hard particle | // |
| | 70°C, RH 60% | white powder (caking) | crystal transformation | pale yellow hard particle | // | pale yellow hard particle | // |
| | 75°C, RH 30% | white powder | crystal transformation | white powder | // | white powder | // |

**Table 6-2 Physical Stability Results of Formulation of API: HPMC-AS-LF (1:2)**

| **Formulation** | **Condition** | **3 days** | | **7 days** | | **18 days** | |
|---|---|---|---|---|---|---|---|
| | | **Appearance** | **Solid-state property** | **Appearance** | **Solid-state property** | **Appearance** | **Solid-state property** |
| F25 | 40°C, RH 60% | white powder | amorphous form | white powder | amorphous form | white powder | amorphous form |
| | 40°C, RH 75% | white powder (caking) | amorphous form | white powder (caking) | amorphous form | white powder (caking) | amorphous form |
| | 60°C, RH 30% | white powder | amorphous form | white powder | amorphous form | white powder | amorphous form |
| | 60°C, RH 75% | white powder (caking) | crystal transformation | pale yellow hard particle | // | pale yellow hard particle | // |
| | 70°C, RH 60% | white powder (caking) | crystal transformation | pale yellow hard particle | // | pale yellow hard particle | // |
| | 75°C, RH 30% | white powder | amorphous form | white powder | crystal transformation | white powder | // |

**Table 6-3 Physical Stability Results of Formulation of API:HPMC-AS-LF (1:3)**

| **Formulation** | **Condition** | **3 days** | | **7 days** | | **18 days** | |
|---|---|---|---|---|---|---|---|
| | | **Appearance** | **Solid-state property** | **Appearance** | **Solid-state property** | **Appearance** | **Solid-state property** |
| F26 | 40°C, RH 60% | white powder | amorphous form | white powder | amorphous form | white powder | amorphous form |
| | 40°C, RH 75% | white powder | amorphous form | white powder (caking) | amorphous form | white powder (caking) | amorphous form |
| | 60°C, RH 30% | white powder | amorphous form | white powder | amorphous form | white powder | amorphous form |
| | 60°C, RH 75% | white powder (caking) | amorphous form | pale yellow hard particle | amorphous form | pale yellow hard particle | amorphous form |
| | 70°C, RH 60% | white powder (caking) | amorphous form | pale yellow hard particle | amorphous form | pale yellow hard particle | amorphous form |
| | 75°C, RH 30% | white powder | amorphous form | white powder | amorphous form | white powder | amorphous form |

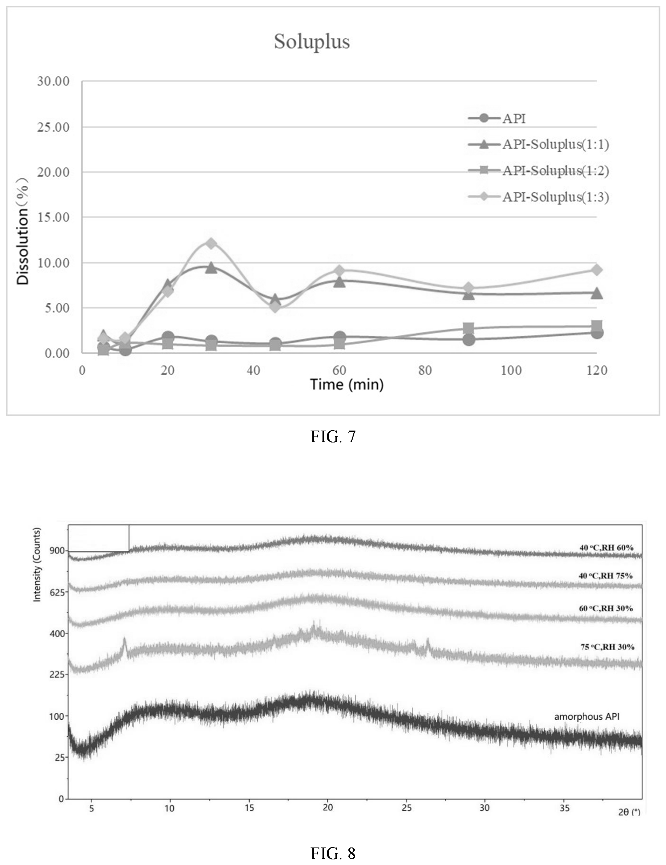

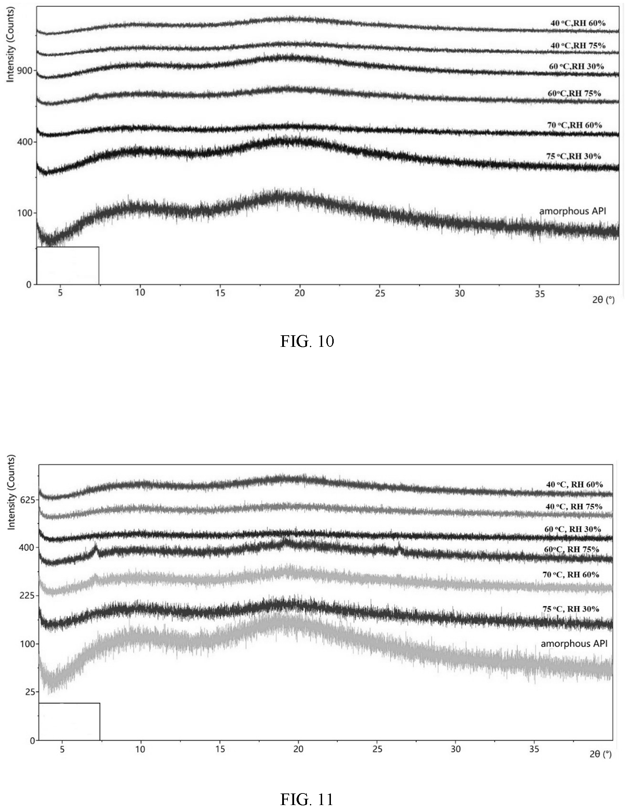

It can be concluded from the above stability test results that, under severe storage conditions (60°C/RH 75%, 70°C/RH 60%, and 75°C/RH 30%), the solid-state properties of Formulation F24 changed at the 3-day time point, and XRD results confirmed crystal transformation. At the 7-day time point, appearance and color change was observed in Formulations F24, F25 and F26. Among them, Formulation F25 (Figure 8) exhibited crystal transformation under 75°C/RH 30%, while no significant changes in solid-state properties were found for Formulation F26 (Figure 10).

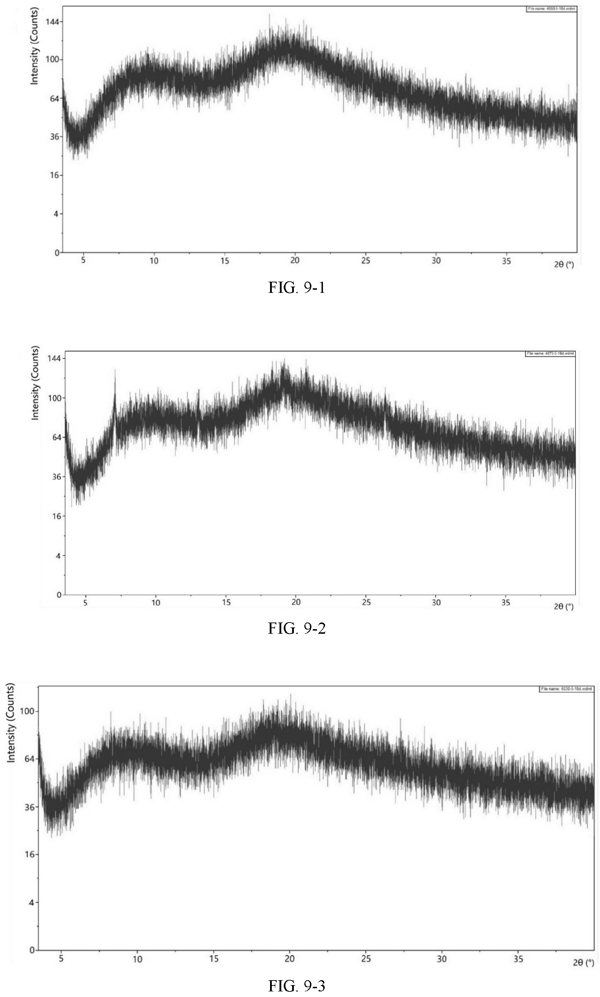

After 18 days of storage under the conditions of 40°C/RH 60%, 40°C/RH 75% and 60°C/RH 30%, Formulation F25 (Figure 9-1 to Figure 9-3) and Formulation F26 (Figure 11) showed no obvious changes in appearance, shape and solid-state property. XRD results verified that both remained as amorphous solid dispersions. Moreover, Formulation F26 (Figure 11) maintained satisfactory stability even after 18 days under extreme conditions of 60°C/RH 75% and 70°C/RH 60%, which supports its feasible and stable industrial production. This indicated that Formulations F25 and F26 possess excellent physical stability at a relatively high drug-to-carrier ratio.

### Test Example 1: Pharmacokinetic assay of the Compound in Rat, Mouse and Canine

For a rat pharmacokinetic assay, male SD rats (180 g to 240 g) were selected and fasted overnight. Three rats were intragastrically administered with 10 mg/kg. The blood of the rats was collected before the administration and at 15 min, 30 min, 1 h, 2 h, 4 h, 8 h, and 24 h after the administration. The blood samples were centrifuged at 4°C for 6 minutes at 8,000 r/min, and plasma was collected and stored at -20°C. Plasma at each time point was taken, and added with 3 to 5 times the amount of an internal standard-containing acetonitrile solution to mix, and the mixture was vortex mixed for 1 minute, and centrifuged at 4°C for 10 minutes at 13,000 r/min. The supernatant was taken and mixed with 3 times the amount of water, and an appropriate amount of the mixed solution was taken for LC-MS/MS analysis. The primary pharmacokinetic parameters were analyzed by WinNonlin 7.0 software non-atrioventricular model.

For a mouse pharmacokinetic assay, male ICR mice (20 g to 25 g) were selected and fasted overnight. Three mice were intragastrically administered with 10 mg/kg. The blood of the mice was collected before the administration and at 15 min, 30 min, 1 h, 2 h, 4 h, 8 h, and 24 h after the administration. The blood samples were 6,800 g and centrifuged at 4°C for 6 minutes, plasma was collected and stored at -80°C. Plasma at each time point was taken, and added with 3-5 times the amount of internal standard-containing acetonitrile to mix, and the mixture was vortex mixed for 1 minute, and centrifuged at 4°C for 10 minutes at 13,000 r/min. The supernatant was mixed with 3 times the amount of water, and an appropriate amount of the mixed solution was taken for LC-MS/MS analysis. The primary pharmacokinetic parameters were analyzed by WinNonlin 7.0 software non-atrioventricular model.

For a canine pharmacokinetic assay, male Beagle dogs (8 kg to 10 kg) were selected and fasted overnight. Three Beagle dogs were intragastrically administered with 5 mg/kg. The blood of the dogs was collected before the administration and at 15 min, 30 min, 1 h, 2 h, 4 h, 8 h, and 24 h after the administration. Another 3 Beagle dogs were intravenously administered with 1 mg/kg. The blood of the dogs was collected before the administration and at 15 min, 30 min, 1 h, 2 h, 4 h, 8 h, and 24 h after the administration. The blood samples were centrifuged at 4°C for 6 minutes at 8,000 r/min, plasma was collected and stored at -20°C. Plasma at each time point was taken, added with 3 to 5 times the amount of internal standard-containing acetonitrile to mix, vortex mixed for 1 minute, and centrifuged at 4°C for 10 minutes at 13,000 r/min. The supernatant was taken and mixed with 3 times the amount of water, and an appropriate amount of the mixed solution was taken for LC-MS/MS analysis. The primary pharmacokinetic parameters were analyzed by WinNonlin 7.0 software non-atrioventricular model.

The results of the rat pharmacokinetic study indicated that, Compounds I-27, I-28, I-29 and I-30 of the present disclosure exhibited good and comparable pharmacokinetic properties.

**Table 7-1 Mouse Pharmacokinetic study of Tested Compounds**

| Compound | Mouse Pharmacokinetic Parameters (intragastric administration) | | | |
|---|---|---|---|---|
| | Cmax (ng/mL) | Tmax (hr) | AUC0-t (h*ng/mL) | T1/2 (hr) |
| I-27 | 3377.5 | 0.50 | 14974.5 | 2.89 |
| I-28 | 2400.2 | 0.50 | 8761.3 | 2.65 |
| I-29 | 4098.9 | 1.00 | 14889.1 | 2.23 |
| I-30 | 2945.5 | 1.00 | 13784.5 | 2.35 |

The results of the mouse pharmacokinetic study indicated that, Compounds I-27, I-28, I-29 and I-30 of the present disclosure exhibited good and comparable pharmacokinetic properties.

**Table 7-2 Pharmacokinetic assay in canine of Tested Compounds**

| Compound | Canine Pharmacokinetic Parameters (intravenous administration) | | | | Canine Pharmacokinetic Parameters (intragastric administration) | | | |
|---|---|---|---|---|---|---|---|---|
| | CL (L/h/kg) | Vz (L/kg) | AUC(0-t) h*ng/mL | T1/2 (h) | Tmax (hr) | Cmax (ng/mL) | AUC(0-t) (h*ng/mL) | T1/2 (hr) |
| I-29 | 0.45 | 5.00 | 2013.9 | 7.64 | 1.00 | 1156.5 | 8730.3 | 7.26 |
| I-30 | 0.26 | 4.88 | 2821.7 | 13.01 | 1.00 | 1025.1 | 12600.9 | 13.37 |

The results indicated that, Compounds I-29 and I-30 of the present disclosure exhibit low clearance following intravenous administration, high systemic exposure via both oral and intravenous routes, and long half-lives. The two compounds present similar pharmacokinetic profiles with good druggability.

### Test Example 2: Thermodynamic Solubility Test

Fasted state simulated gastric fluid (FaSSGF, pH 1.6, 80µM sodium taurocholate, 20µM lecithin, 0.1g pepsin and 34.2mM sodium chloride per liter of solution), fasted state simulated intestinal fluid (FaSSIF, pH 6.5, 3mM sodium taurocholate, 0.2mM lecithin, 38.4 mM sodium hydroxide, 68.62mM sodium chloride and 19.12mM maleic acid per liter of solution) and phosphate buffered saline (PBS, pH 7.4, 100mM phosphate buffer, 11g disodium hydrogen carbonate and 3.5g sodium dihydrogen phosphate dihydrate per liter of solution) as test buffers were prepared.

The compound was accurately weighed and diluted with the above test buffers of different pH values to prepare a working solution at 4 mg/mL. The mixture was shaken at 1000 rpm for 1 h and equilibrated at room temperature overnight. Thereafter, the sample was centrifuged at 12000 rpm for 10 min to remove undissolved particles. The supernatant was transferred into a new centrifuge tube, and the compound concentration in the supernatant was determined by LC-MS/MS.

The results indicated that, Compounds I-27, I-28, I-29 and I-30 all exhibited good solubility.

### Test Example 3: Pharmacokinetic assay in canine of SDD Formulations

For a canine pharmacokinetic assay, male Beagle dogs (8 kg to 10 kg) were selected and fasted overnight. Nine Beagle dogs were randomly divided into three groups, with three animals in each group. Formulations prepared with respective formulations of the present application were administered to each group via oral gavage at a dose of 20 mg/kg. The blood of the dogs approximately 0.5mL was collected before the administration and at 15min, 1 h, 2 h, 4 h, 8 h and 24 h after administration, and transferred into anticoagulant tubes containing EDTA-K₂. The blood samples were centrifuged at 4 °C for 10 min at 1500g to separate plasma. After sample pretreatment by acetonitrile protein precipitation, an appropriate amount of the mixed solution was taken for LC-MS/MS analysis. The primary pharmacokinetic parameters were analyzed by WinNonlin 7.0 software non-atrioventricular model. The results are shown in Table 7-3.

**Table 7-3 Canine Pharmacokinetic Parameters (Intragastric administration)**

| Tested Formulation | Dog Pharmacokinetic Parameters (intragastric administration) | | | |
|---|---|---|---|---|
| | Tmax (hr) | Cmax (ng/mL) | AUC(0-t) (h*ng/mL) | T1/2 (hr) |
| API:HPMC-AS-LF 1:3 | 12.0 | 1390 | 20000 | 12.6 |

Based on the canine pharmacokinetic results, the SDD formulation with an API to HPMC-AS-LF mass ratio of 1:3 presents excellent systemic exposure at a high administered dose. This formulation can maintain a supersaturated state for a prolonged period and sustain stable and continuous in vivo absorption beyond 4 hours after dosing.

The embodiments of the present disclosure have been described above. However, the present disclosure is not limited to the foregoing embodiments. Any modification, equivalent replacement, improvement and the like made within the spirit and principle of the present invention shall fall within the protection scope of the present invention.

## Claims

1. A solid dispersion of a P2X3 receptor antagonist, wherein the solid dispersion comprises a P2X3 receptor antagonist and a pharmaceutically acceptable carrier;
wherein, the P2X3 receptor antagonist is selected from a compound represented by Formula I, or a tautomer, a stereoisomer, a hydrate, a solvate, a pharmaceutically acceptable salt, or a prodrug thereof: 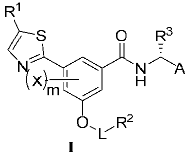
wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
X is halogen;
m is an integer selected from 1, 2, or 3;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R³ is independently selected from hydrogen, unsubstituted C₁-C₄ alkyl, or C₁-C₄ alkyl substituted with 1 to 5 identical or different halogen atoms;
R⁴ is independently selected from hydrogen or C₁-C₄ alkyl;
R^{b} and R^{c} are independently selected from hydrogen or C₁-C₄ alkyl; and
A is independently unsubstituted or R^{e}-substituted 5-10-membered heteroaryl, wherein the R^{e}-substituted 5-10-membered heteroaryl has one or more R^{e} substituents, each R^{e} substituent being independently selected from halogen, unsubstituted C₁-C₃ alkyl, C₁-C₃ alkyl substituted with 1 to 5 identical or different halogen atoms, unsubstituted -O-(C₁-C₃ alkyl), or -O-(C₁-C₃ alkyl) substituted with 1 to 5 identical or different halogen atoms; when the one or more R^{e} substituents include a plurality of R^{e} substituents, the plurality of R^{e} substituents is identical or different.

2. The solid dispersion according to claim 1, wherein the solid dispersion is an amorphous solid dispersion;
preferably, the P2X3 receptor antagonist is present in an amorphous form;
preferably, the pharmaceutically acceptable carrier is a pharmaceutically acceptable polymeric carrier;
preferably, the pharmaceutically acceptable carrier is selected from any one or more of the following:
1) a cellulose derivative carrier, for example, a hypromellose E5, a hypromellose acetate succinate LF, a hydroxypropyl cellulose, or a hypromellose phthalate;
2) a polyacrylic resin carrier, for example, a Eudragit L100-55, a Eudragit S 100, a Eudragit L100;
3) a vinyl polymer carrier, for example, a polyvinylcaprolactam-polyvinyl acetate-polyethylene glycol graft copolymer, a copovidone VA64, or a copovidone S630;
preferably, the pharmaceutically acceptable carrier is selected from any one or more of the following: hypromellose HPMC, copovidone PVP, hypromellose acetate succinate HPMC-AS, polymethacrylate, and polyvinylcaprolactam-polyvinyl acetate-polyethylene glycol graft copolymer; preferably, it is selected from any one or more of the following: hypromellose HPMC, hypromellose acetate succinate HPMC-AS, and polymethacrylate;
preferably, the pharmaceutically acceptable carrier is selected from any one or more of the following: hypromellose E5, copovidone VA64, hypromellose acetate succinate LF, Eudragit L100-55, and polyvinylcaprolactam-polyvinyl acetate-polyethylene glycol graft copolymer; preferably, it is selected from any one or more of the following: hypromellose E5, hypromellose acetate succinate LF, and Eudragit L100-55.

3. The solid dispersion according to claim 1 or 2, wherein the mass ratio of the P2X3 receptor antagonist to the pharmaceutically acceptable carrier is 1:(0.1-10), preferably 1:(0.5-5), for example, 1:0.5, 1:1, 1:2, 1:3, 1:4 or 1:5;
preferably, the solid dispersion comprises the following components:
the P2X3 receptor antagonist: 10wt%-60wt%
the pharmaceutically acceptable carrier: 40wt%-90wt%;
preferably, the solid dispersion comprises the following components:
the P2X3 receptor antagonist: 20wt%-55wt%
the pharmaceutically acceptable carrier: 45wt%-80wt%;
preferably, the solid dispersion comprises the following components:
the P2X3 receptor antagonist: 25wt%-50wt% (e.g., 25wt%, 33wt%, 33.3wt%, 50wt%)
the pharmaceutically acceptable carrier: 50wt%-75wt% (e.g., 50wt%, 67wt%, 66.7wt%, 75wt%);
preferably, the solid dispersion comprises the following components: the P2X3 receptor antagonist and the pharmaceutically acceptable carrier, wherein the content of the pharmaceutically acceptable carrier is not less than 66.7wt%;
preferably, the solid dispersion comprises the following components: the P2X3 receptor antagonist and the pharmaceutically acceptable carrier, wherein the content of the pharmaceutically acceptable carrier is not less than 75wt%;
preferably, the solid dispersion comprises the following components:
the P2X3 receptor antagonist: 25 wt%, 33.3 wt%, 50 wt%
the pharmaceutically acceptable carrier: 50 wt%, 66.7 wt%, 75 wt%;
the pharmaceutically acceptable carrier is selected from hypromellose E5, hypromellose acetate succinate LF, and Eudragit L100-55;
preferably, the solid dispersion comprises the following components:
the P2X3 receptor antagonist: 25 wt%
the pharmaceutically acceptable carrier: 75 wt%;
the pharmaceutically acceptable carrier is selected from hypromellose E5, hypromellose acetate succinate LF, and Eudragit L100-55;
preferably, the solid dispersion comprises the following components:
the P2X3 receptor antagonist: 33.3 wt%
the pharmaceutically acceptable carrier: 66.7 wt%;
the pharmaceutically acceptable carrier is selected from hypromellose E5, hypromellose acetate succinate LF, and Eudragit L100-55;
preferably, the solid dispersion comprises the following components: the P2X3 receptor antagonist and hypromellose acetate succinate LF, wherein the content of hypromellose acetate succinate LF is not less than 75%;
preferably, the solid dispersion comprises the following components:
the P2X3 receptor antagonist: 25 wt%
hypromellose acetate succinate LF: 75 wt%;
preferably, the solid dispersion comprises the following components: the P2X3 receptor antagonist and hypromellose acetate succinate LF, wherein the content of hypromellose acetate succinate LF is not less than 66.7 wt%;
preferably, the solid dispersion comprises the following components:
the P2X3 receptor antagonist: 33.3 wt%
hypromellose acetate succinate LF: 66.7 wt%;
preferably, the solid dispersion optionally further comprises a solvent;
preferably, the solvent is a volatile solvent;
preferably, the solvent is selected from any one or more of the following: dichloromethane, ethanol, acetone, and methanol; preferably acetone, or mixed solvent of the dichloromethane and methanol;
preferably, the solid dispersion optionally further comprises any one or more of the following: a sweetener, a flavoring agent, a colorant, a surfactant, a filler, a lubricant, a disintegrant, a disintegration accelerator, a recrystallization inhibitor, a defoamer, an antioxidant and a pH regulator.

4. The solid dispersion according to any one of preceding claims 1 to 3, wherein: the compound represented by Formula I, or the tautomer, the stereoisomer, the hydrate, the solvate, the pharmaceutically acceptable salt, or the prodrug thereof is: 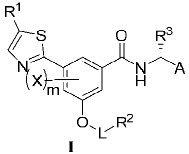 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
X is halogen;
m is an integer selected from 1, 2, or 3;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents each independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R³ is independently selected from hydrogen, unsubstituted C₁-C₄ alkyl, or C₁-C₄ alkyl substituted with 1 to 5 identical or different halogen atoms;
R⁴ is independently selected from hydrogen or C₁-C₄ alkyl;
R^{b} and R^{c} are independently selected from hydrogen or C₁-C₄ alkyl; and
A is independently unsubstituted or R^{e}-substituted 5-10-membered heteroaryl, wherein the R^{e}-substituted 5-10-membered heteroaryl has one or more R^{e} substituents, each R^{e} substituent being independently selected from halogen, unsubstituted C₁-C₃ alkyl, C₁-C₃ alkyl substituted with 1 to 5 identical or different halogen atoms, unsubstituted -O-(C₁-C₃ alkyl), or -O-(C₁-C₃ alkyl) substituted with 1 to 5 identical or different halogen atoms; when the one or more R^{e} substituents include a plurality of R^{e} substituents, the plurality of R^{e} substituents is identical or different;
preferably, when R¹ is halogen, the halogen is F, Cl, Br, or I, and preferably Cl;
preferably, when R¹ is the unsubstituted C₁-C₄ alkyl, the C₁-C₄ alkyl is methyl, ethyl, n-propyl, or isopropyl, and preferably methyl or ethyl;
preferably, when R¹ is the C₁-C₄ alkyl substituted with 1 to 5 identical or different halogen atoms, the C₁-C₄ alkyl is methyl, ethyl, n-propyl, or isopropyl, and preferably methyl or ethyl;
preferably, when R¹ is the C₁-C₄ alkyl substituted with 1 to 5 identical or different halogen atoms, the halogen atoms are F, Cl, Br, or I, and preferably Cl or F;
preferably, when X is halogen, the halogen is F or Cl;
preferably, m is an integer selected from 1, 2 or 3, and preferably 1;
preferably, when L is -(CH₂)ₙ-, n is an integer of 0, 1, or 2, and preferably n is 0 or 1;
preferably, when R² is the unsubstituted or R^{a}-substituted C₁-C₆ alkyl, the C₁-C₆ alkyl is C₁-C₄ alkyl, and preferably sec-butyl;
preferably, when R² is the unsubstituted or R^{a}-substituted C₁-C₆ alkyl, the C₁-C₆ alkyl is preferably methyl, ethyl, n-propyl, isopropyl, n-butyl, isobutyl, sec-butyl, or tert-butyl;
preferably, when R² is the unsubstituted or R^{a}-substituted C₃-C₇ cycloalkyl, the C₃-C₇ alkyl is cyclopropyl, cyclobutyl, cyclopentyl, or cyclohexyl, and preferably cyclopropyl;
preferably, when R² is the unsubstituted or R^{a}-substituted 4-7-membered heterocycloalkyl, the 4-7-membered heterocycloalkyl is 4-membered, 5-membered, or 6-membered heterocycloalkyl;
preferably, when R² is the unsubstituted or R^{a}-substituted 4-7-membered heterocycloalkyl, the 4-7-membered heterocycloalkyl has one or more heteroatoms selected from N, S, O, or P, and preferably selected from N or O;
preferably, when R² is the unsubstituted or R^{a}-substituted 4-7-membered heterocycloalkyl, the 4-7-membered heterocycloalkyl has 1-3 heteroatoms, and preferably 1 or 2 heteroatoms;
preferably, when R² is the unsubstituted or R^{a}-substituted 4-7-membered heterocycloalkyl, R² has 1-3 R^{a} substituents, and preferably one R^{a} substituent;
preferably, R^{a} is hydroxyl;
preferably, when R^{a} is halogen, the halogen is F, Cl, Br, or I, and preferably F or Cl;
preferably, when R^{a} is C₁-C₆ alkyl, the C₁-C₆ alkyl is C₁-C₃ alkyl, and preferably methyl;
preferably, when R^{a} is the deuterated C₁-C₆ alkyl, the C₁-C₆ alkyl is deuterated C₁-C₃ alkyl, and preferably 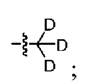
preferably, when R^{a} is -(C₁-C₆ alkylene)-OH, the C₁-C₆ alkylene is C₁-C₄ alkylene, preferably methylene, ethylene, n-propylene, or isopropylidene, and more preferably methylene;
preferably, R³ is hydrogen;
preferably, when R³ is the unsubstituted C₁-C₄ alkyl, the C₁-C₄ alkyl is methyl, ethyl, n-propyl, or isopropyl, and preferably methyl;
preferably, when A is the R^{e}-substituted 5-10-membered heteroaryl, the 5-10-membered heteroaryl is 6-membered heteroaryl, and preferably pyrimidinyl or pyridazinyl;
preferably, when A is the R^{e}-substituted 5-10-membered heteroaryl, it is mono-substituted;
preferably, when A is the R^{e}-substituted 5-10-membered heteroaryl, the R^{e} substituent is C₁-C₃ alkyl substituted with 1 to 5 identical or difference halogen atoms, and preferably trifluoromethyl;
preferably, when A is the R^{e}-substituted 5-10-membered heteroaryl, the R^{e} substituent is unsubstituted C₁-C₃ alkyl, and preferably methyl;
preferably, -L-R² is selected from 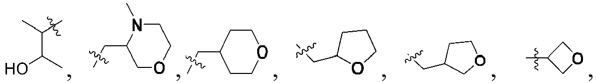 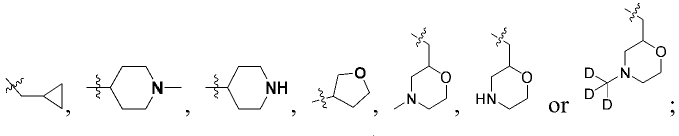
preferably, -L-R² is 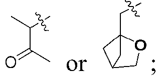
preferably, -L-R² is selected from 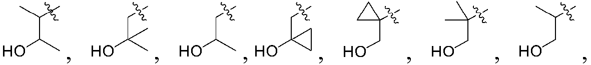 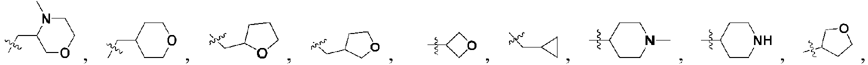 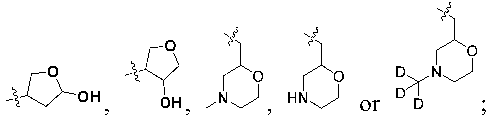
preferably, R¹ is methyl, ethyl, or Cl;
preferably, -L-R² is selected from 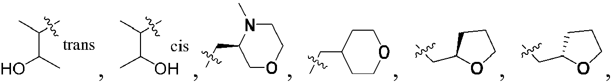 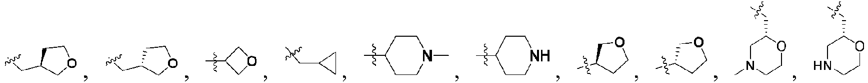 or 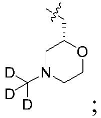
preferably, -L-R² is selected from 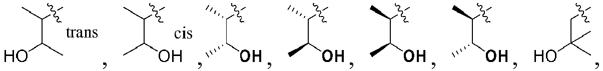 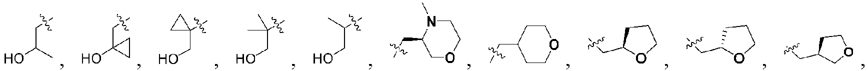 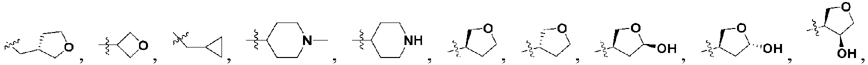 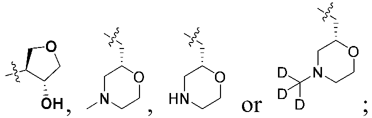
preferably, R³ is methyl or hydrogen;
preferably, A is 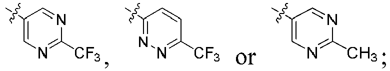
preferably, the compound represented by Formula I, or the tautomer, the stereoisomer, the hydrate, the solvate, the pharmaceutically acceptable salt, or the prodrug thereof is: 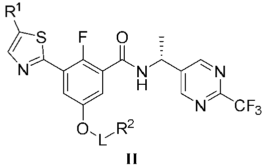
wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R⁴ is independently selected from hydrogen or C₁-C₄ alkyl;
R^{b} and R^{c} are independently selected from hydrogen or C₁-C₄ alkyl;
preferably, the compound represented by Formula I, or the tautomer, the stereoisomer, the hydrate, the solvate, the pharmaceutically acceptable salt, or the prodrug thereof is: 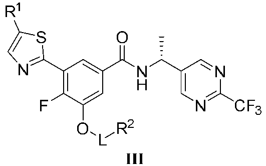
wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R⁴ is independently selected from hydrogen or C₁-C₄ alkyl;
R^{b} and R^{c} are independently selected from hydrogen or C₁-C₄ alkyl;
preferably, the compound represented by Formula I, or the tautomer, the stereoisomer, the hydrate, the solvate, the pharmaceutically acceptable salt, or the prodrug thereof is: 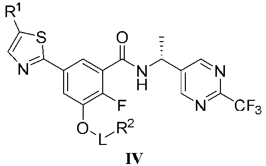
wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R⁴ is independently selected from hydrogen or C₁-C₄ alkyl;
R^{b} and R^{c} are independently selected from hydrogen or C₁-C₄ alkyl;
preferably, the compound represented by Formula I, or the tautomer, the stereoisomer, the hydrate, the solvate, the pharmaceutically acceptable salt, or the prodrug thereof is: 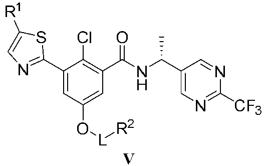
wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R⁴ is independently selected from hydrogen or C₁-C₄ alkyl;
R^{b} and R^{c} are independently selected from hydrogen or C₁-C₄ alkyl;
preferably, the compound represented by Formula I, or the tautomer, the stereoisomer, the hydrate, the solvate, the pharmaceutically acceptable salt, or the prodrug thereof is: 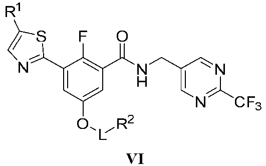
wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R⁴ is independently selected from hydrogen or C₁-C₄ alkyl;
R^{b} and R^{c} are independently selected from hydrogen or C₁-C₄ alkyl;
preferably, the compound represented by Formula I, or the tautomer, the stereoisomer, the hydrate, the solvate, the pharmaceutically acceptable salt, or the prodrug thereof is: 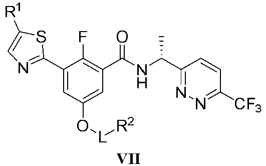
wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R⁴ is independently selected from hydrogen or C₁-C₄ alkyl;
preferably, the compound represented by Formula I, or the tautomer, the stereoisomer, the hydrate, the solvate, the pharmaceutically acceptable salt, or the prodrug thereof is: 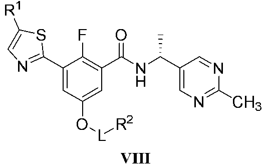
wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted 5-8-membered aryl, unsubstituted or R^{a}-substituted 5-10-membered heteroaryl, unsubstituted or R^{a}-substituted 4-7-membered heterocycloalkyl, or unsubstituted or R^{a}-substituted 6-12-membered heterobicycloalkyl, wherein the R^{a}-substituted C₁-C₆ alkyl, the R^{a}-substituted C₃-C₇ cycloalkyl, the R^{a}-substituted 5-8-membered aryl, the R^{a}-substituted 5-10-membered heteroaryl, the R^{a}-substituted 4-7-membered heterocycloalkyl, or the R^{a}-substituted 6-12-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
the unsubstituted or R^{a}-substituted 5-10-membered heteroaryl has 1 to 3 heteroatoms selected from one or more of N, S, O, or P; the unsubstituted or R^{a}-substituted 4-7-membered heterocycloalkyl has 1 to 3 heteroatoms selected from one or more of N, S, O, or P; and the unsubstituted or R^{a}-substituted 6-12-membered heterobicycloalkyl has 1 to 3 heteroatoms selected from one or more of N, S, O, or P;
R⁴ is independently selected from hydrogen or C₁-C₄ alkyl;
R^{b} and R^{c} are independently selected from hydrogen or C₁-C₄ alkyl.

5. The solid dispersion according to any one of preceding claims 1 to 4, wherein the compound represented by Formula I, or the tautomer, the stereoisomer, the hydrate, the solvate, the pharmaceutically acceptable salt, or the prodrug thereof is selected from:

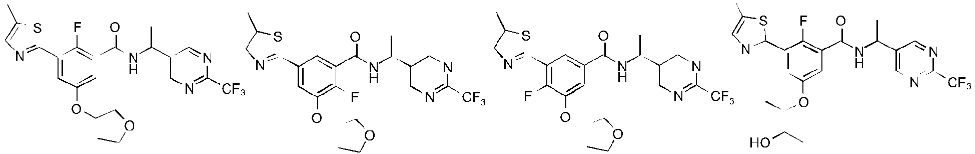

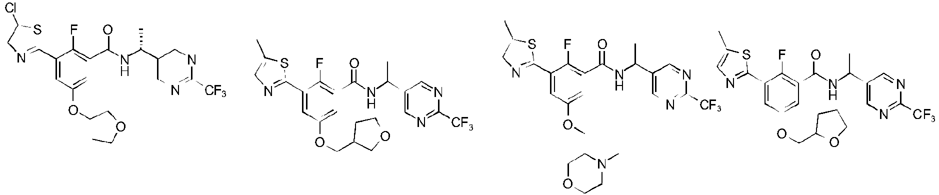

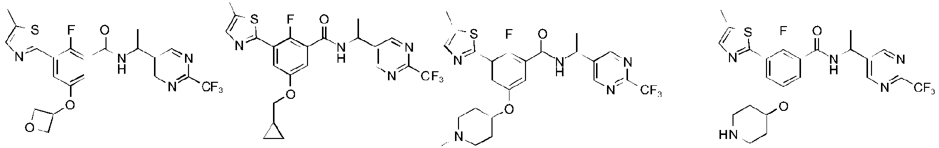

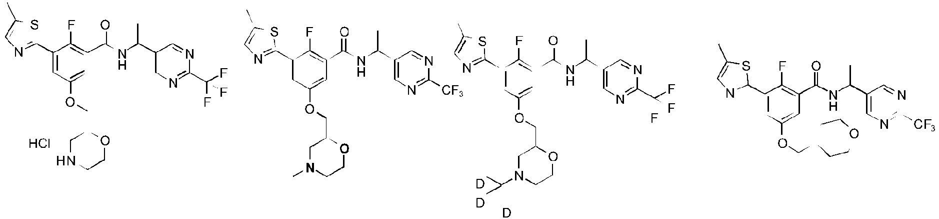

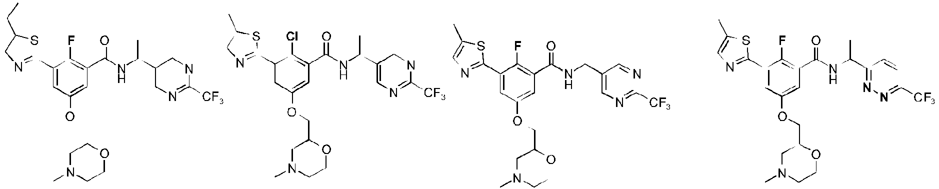

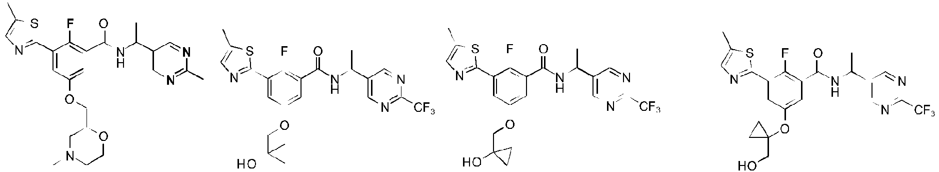

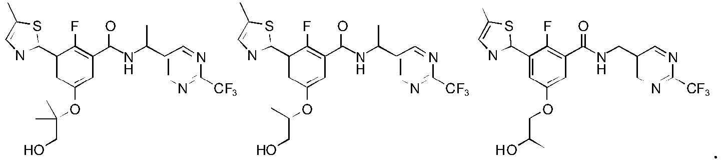

6. The solid dispersion according to any one of preceding claims 1 to 4, wherein the compound represented by Formula I, or the tautomer, the stereoisomer, the hydrate, the solvate, the pharmaceutically acceptable salt, or the prodrug thereof is selected from: 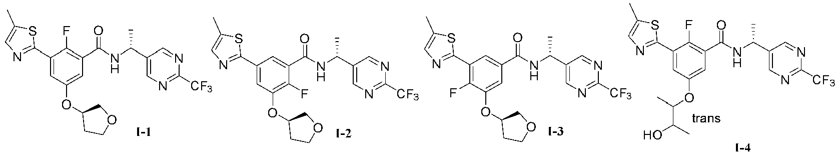 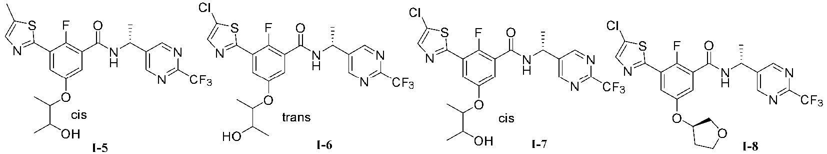 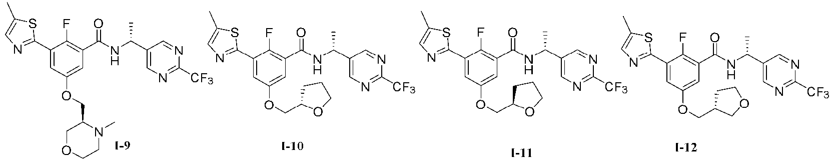 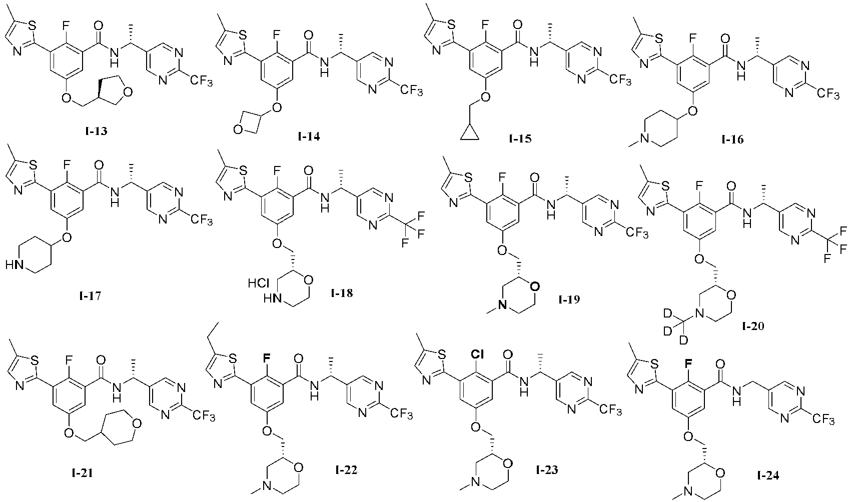 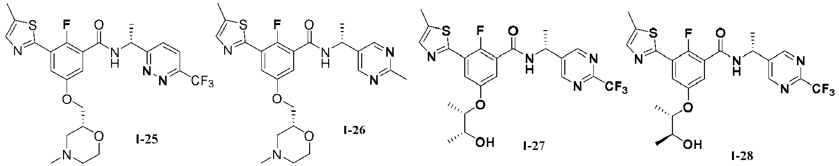 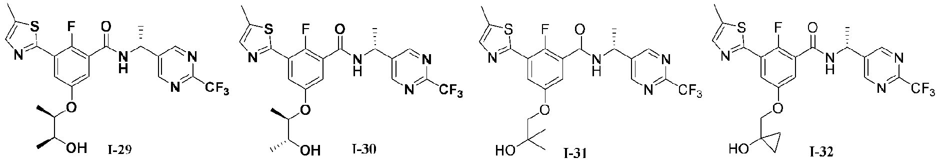 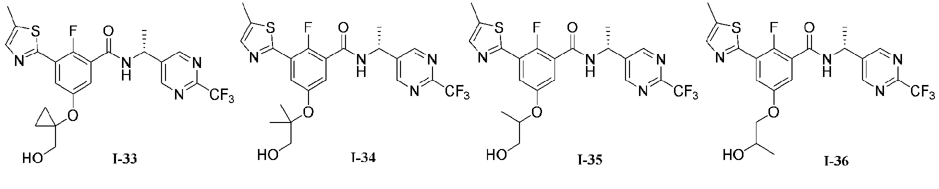 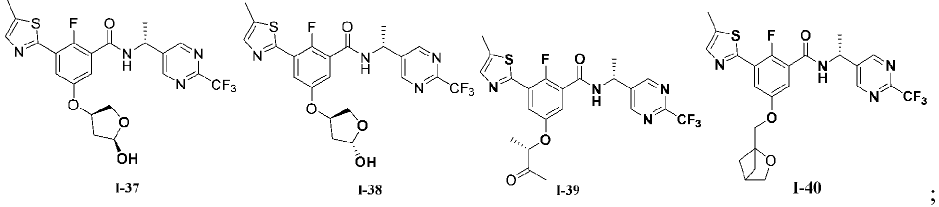 preferably, the P2X3 receptor antagonist is selected from a compound represented by Formula I (Formula I-27), or a tautomer, a stereoisomer, a hydrate, a solvate, a pharmaceutically acceptable salt, or a prodrug thereof:

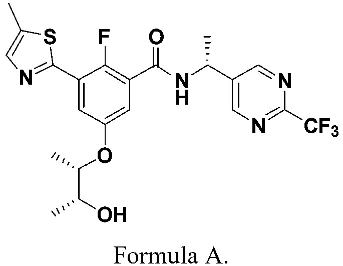

7. A process for preparing the solid dispersion according to any one of preceding claims 1 to 6, which comprises the following steps:
(1) Adding each component to a solvent and stirring to dissolve;
(2) Subjecting the dissolved solution to a drying treatment to obtain the solid dispersion;
preferably, in step (1), the process is specifically: first adding the pharmaceutically acceptable carrier to the solvent and stirring to dissolve; then adding the P2X3 receptor antagonist and continuing to stir to dissolve;
preferably, in step (2), the drying treatment method is spray drying;
preferably, in step (2), the process is specifically: performing pressure filtration on the dissolved solution into a transfer tank, then drying the pressure-filtered solution to obtain the solid dispersion;
preferably, performing pressure filtration into the transfer tank through a precision filter with a specific pore size;
preferably, after the drying treatment in step (2), the dried solid dispersion is optionally further subjected to reduced pressure drying;
preferably, in step (1), the stirring time is not less than 60 min (e.g., not less than 120 min); preferably 60-120 min or 120-180 min;
preferably, in step (2), the pore size of the precision filter is 1µm - 22µm, preferably 5µm;
preferably, in step (2), the feeding frequency of the spray drying is 5Hz -50 Hz, preferably 10Hz - 20Hz;
and/or, atomization pressure is 0.1MPa - 1.0MPa, preferably 0.1MPa - 0.2MPa;
and/or, inlet air temperature is 60°C-120°C, preferably 85°C-110°C;
and/or, outlet air temperature is 40°C-70°C, preferably 50°C-55°C.

8. A pharmaceutical composition, wherein the pharmaceutical composition comprises the solid dispersion according to any one of preceding claims 1 to 6 and the pharmaceutically acceptable carrier, a diluent or an excipient.

9. Use of the solid dispersion according to any one of preceding claims 1 to 6, or the pharmaceutical composition according to claim 8 in preparation of a medicament for the treatment and/or prevention of P2X3-associated diseases; wherein,
preferably, the P2X3-associated diseases are pain, respiratory system diseases or genitourinary system diseases;
preferably, the pain is chronic pain, acute pain, endometriosis-associated pain, neuropathic pain, back pain, cancer pain, inflammatory pain, postoperative pain, migraine or visceral pain;
and/or, the genitourinary system diseases are reduced bladder capacity, frequent micturition, urge incontinence, stress incontinence, bladder hyperreactivity, benign prostatic hyperplasia, prostatitis, detrusor hyperreflexia, pollakiuria, nocturia, urgency, overactive bladder, pelvic hypersensitivity, urethritis, pelvic pain syndrome, prostatodynia, or cystitis;
and/or, the respiratory system diseases are chronic obstructive pulmonary disease, pulmonary arterial hypertension, pulmonary fibrosis, asthma and obstructive sleep apnea, chronic cough, refractory chronic cough or acute cough.

10. A method for the treatment and/or prevention of P2X3-associated diseases, which comprises administering an effective amount of the solid dispersion according to any one of preceding claims 1 to 6, pharmaceutical composition according to claim 8 to a patient suffering from P2X3-associated diseases.
preferably, the P2X3-associated diseases include pain, respiratory system diseases or genitourinary system diseases;
Preferably, the pain includes chronic pain, acute pain, endometriosis-associated pain, neuropathic pain, back pain, cancer pain, inflammatory pain, postoperative pain, migraine or visceral pain;
and/or, the genitourinary system diseases include reduced bladder capacity, frequent micturition, urge incontinence, stress incontinence, bladder hyperreactivity, benign prostatic hyperplasia, prostatitis, detrusor hyperreflexia, pollakiuria, nocturia, urgency, overactive bladder, pelvic hypersensitivity, urethritis, pelvic pain syndrome, prostatodynia, or cystitis;
and/or, the respiratory system diseases include chronic obstructive pulmonary disease, pulmonary arterial hypertension, pulmonary fibrosis, asthma and obstructive sleep apnea, chronic cough, refractory chronic cough or acute cough.
